# EUROPEAN PATENT SPECIFICATION

(11) **EP 1 695 255 B1**
(45) Date of publication and mention of the grant of the patent: **07.09.2016**
(21) Application number: 04815129.4
(22) Date of filing: 20.12.2004
(51) Int. Cl.: G06F 19/00

(54) **METHODS AND SYSTEMS FOR PROTEIN AND PEPTIDE EVIDENCE ASSEMBLY**
VERFAHREN UND SYSTEME ZUR PROTEIN- UND PEPTID-INDIZIEN-ZUSAMMENSTELLUNG
PROCEDES ET SYSTEMES DE MISE EN EVIDENCE DE PROTEINES ET DE PEPTIDES

(30) Priority: 19.12.2003 US 531405 P; 05.08.2004 US 599321 P
(43) Date of publication of application: 30.08.2006
(73) Proprietor: Applied Biosystems, LLC, Carlsbad, CA 92008 (US)
(72) Inventor: SEYMOUR, Sean, Berkeley, California 94709 (US); LOBODA, Alex, Belmont, California 94002 (US); TANG, Wilfred, Albany, CA 94706 (US)
(74) Representative: Roberts, Mark Peter
(86) International application number: PCT/US2004/043013
(87) International publication number: WO 2005/064345

(56) References cited:
- NESVIZHSKII ALEXEY I ET AL: "A statistical model for identifying proteins by tandem mass spectrometry." ANALYTICAL CHEMISTRY. 1 SEP 2003, vol. 75, no. 17, 1 September 2003 (2003-09-01), pages 4646-4658, XP002349273 ISSN: 0003-2700
- ZHANG W ET AL: "Profound: An expert system for protein identification using mass spectrometric peptide mapping information" ANALYTICAL CHEMISTRY, AMERICAN CHEMICAL SOCIETY. COLUMBUS, US, vol. 72, no. 11, 1 June 2000 (2000-06-01), pages 2482-2489, XP002902847 ISSN: 0003-2700
- JENSEN O N ET AL: "IDENTIFICATION OF THE COMPONENTS OF SIMPLE PROTEIN MIXTURES BY HIGH-ACCURACY PEPTIDE MASS MAPPING AND DATABASE SEARCHING" ANALYTICAL CHEMISTRY, AMERICAN CHEMICAL SOCIETY. COLUMBUS, US, vol. 69, no. 23, 1 December 1997 (1997-12-01), pages 4741-4750, XP001154861 ISSN: 0003-2700
- Brian C. Searle: "Scaffold: A bioinformatic tool for validating MS/MS-based proteomic studies", PROTEOMICS, vol. 10, no. 6, 13 March 2010 (2010-03-13) , pages 1265-1269, XP055206340, ISSN: 1615-9853, DOI: 10.1002/pmic.200900437

## Description

The present disclosure generally relates to methods, and systems for the identification and quantitation of proteins and peptides via mass spectrometry.

Protein identification is commonly performed by reducing a mixture of protein often enzymatically - to smaller peptides. The peptides are typically subjected to instrument analysis (often via chromatography and mass spectrometry) and various levels of informatics analysis to determine the identity of whole or partial peptides. The set of putatively identified peptides can then be assembled into evidence to support the presence of proteins in a sample. Other strategies include analysis of intact proteins with various analytical techniques. Some variants of this approach can break proteins into smaller segments that are analyzed individually, resulting in a similar assembly of peptide segments into evidence to support the identification of full proteins.

Often, identification of peptides and proteins is performed by consulting databases of proteins, DNA, or RNA sequences. Segments of full sequences can be used to develop hypotheses for the identity of analyzed peptides. Often, many whole or partial peptide sequences can appear in several different proteins. Also, because databases of proteins and genetic sequences are imperfect, sequence segments may appear in many database entries due to errant redundancy. Hypotheses for the identification of peptides may also be derived without the benefit of consulting a database-for example, using *de novo* sequencing.

Often, when database-driven methods are used for searching, establishing association of a peptide sequence with its parent protein Is trivial; when databases are not used during search, this protein association can be established by comparison of alignment to a database of macromolecules. Because of similarity among protein sequences, peptide sequences of varying lengths from different proteins may be considered as reasonable hypotheses for the identity of a peptide molecule. Defining a "peptide match" to be a hypothesis for the identity or partial identity of an analyzed peptide molecule, uncertainty about which of many matches to an analysis of a peptide is correct, if any, can lead to uncertainty in which protein is supported. Even if the choice of best peptide match is clear, there may still be uncertainty at the protein level. For example, a user might find three glycogen phosphorylases in the protein list and thus be led to believe that all three proteins are present in the sample when in fact they are not. In some cases a multiplicity of similar proteins may only be a manifestation of the fact that the peptides identified by the instrument are common to each of the three proteins. However, in some cases, all three proteins may in fact be present. To more accurately determine the presence of a protein, the user must rely on additional evidence to either support the presence, or cause the removal, of a protein in the list. This type of analysis often requires a tedious comparison of the peptides associated with similar proteins to determine which peptides are not common among the proteins and whether these constitute sufficient evidence to justify declaring the presence of more than one variation of the protein. Methods to mitigate this effort and produce a statistically valid declaration of present proteins can be useful in areas such as protein identification, drug discovery, protein and gene expression, biomarkers, and other areas of systems biology.

Nesvizhskii, Alexey I. et al: "A statistical model for identifying proteins by tandem mass spectrometry", Anal. Chem., vol. 75, no. 17, pp 4646-4658 discloses obtaining MS/MS spectra, scoring possible peptide hypotheses and finding the best match peptide.

Jensen O. N. et al: "Identification of the components of simple protein mixtures by high-accuracy peptide mass mapping and database searching", Anal. Chem., vol. 69, no. 23, pp 4741-4750 discloses alternative peptide mass mapping.

The invention is defined in the claims.

Some embodiments of the present teachings provide a method and apparatus to mitigate manual examination of protein lists by making the a priori assumption that only one form of a protein is present. Additional evidence can be used to establish if more than one form is present. Various embodiments permit the user to control the level of evidence required before declaring that more than one form of a protein is present. Various embodiments also provide a protein group viewer that permits easy visualization of peptides-to-protein associations and differences in the supporting evidence for similar proteins.

The skilled artisan will understand that the drawings, described below, are for illustration purposes only. The drawings are not intended to limit the scope of the present teachings in any way.
Figure 1 illustrates a typical protein identification workflow where proteins are digested to form peptides, injected into a mass spectrometer and peptides are identified. Subsequently, peptides are compared to a database of proteins to determine the proteins present in the sample.
Figure 2 illustrates enzymatic digestion of a protein via trypsin.
Figure 3 demonstrates the principle that peptides can map to more than one protein.
Figure 4 demonstrates how two forms of a related protein can possess distinct peptides that can differentiate one protein from the other.
Figure 5 shows an embodiment of typical protein database search results where multiple forms of a protein are reported when it is likely that only one form is in the sample.

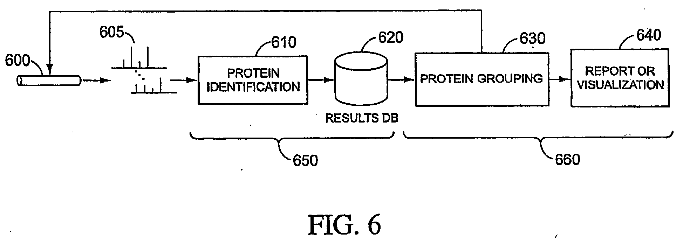

Figure 6 illustrates an embodiment of the present teachings that can be used for protein identification.
Figure 7 illustrates how various embodiments of the present teaching use overlapping peptide evidence to group related proteins.
Figure 8 demonstrates how multiple peptide hypotheses from one spectrum can be used as evidence for the presence of several proteins.

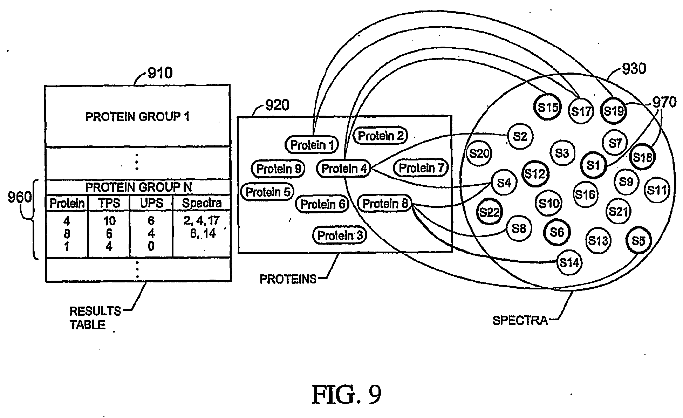

Figure 9 shows how some embodiments of the present teachings assume that one spectrum can only lead to one correct peptide hypothesis, thus once the most probable peptide hypothesis is determined, future peptide hypotheses are not permitted to use that same spectrum.
Figure 10 illustrates how some embodiments of the teachings, reduce false positive protein identification by considering the effects of protein modifications.
Figure 11 shows various ways the present teachings can visually represent protein groups.
Figure 12 illustrates how some embodiments of the present teachings receive a list of putative proteins, groups them and identifies winners in each group.
Figure 13 shows an embodiment of the present teachings that relates protein summary information to the user.
Figure 14 shows an embodiment of the present teachings that relates peptide summary information to the user.

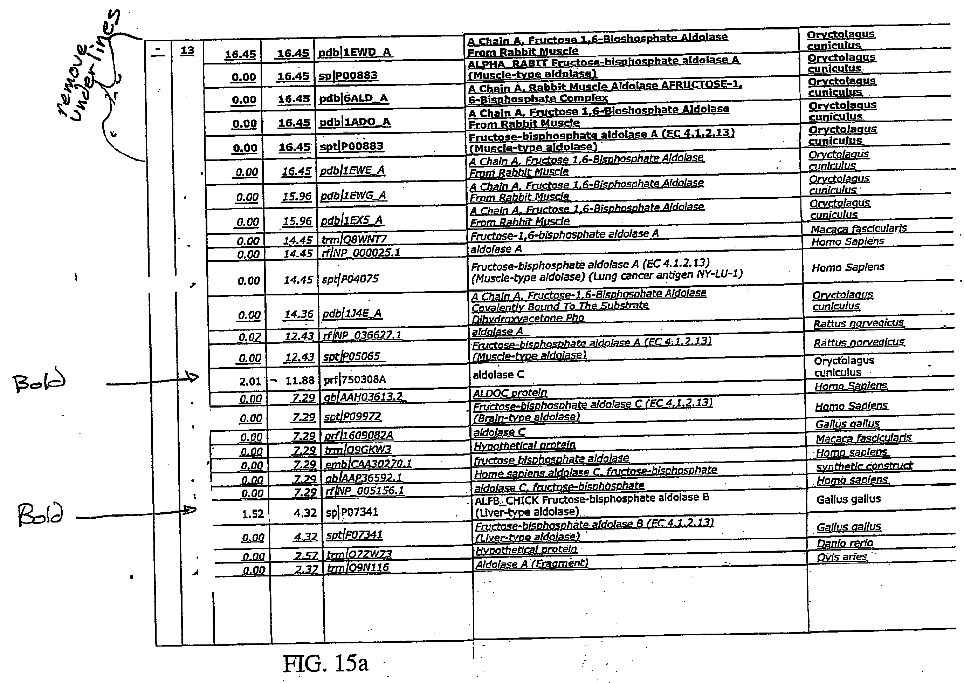

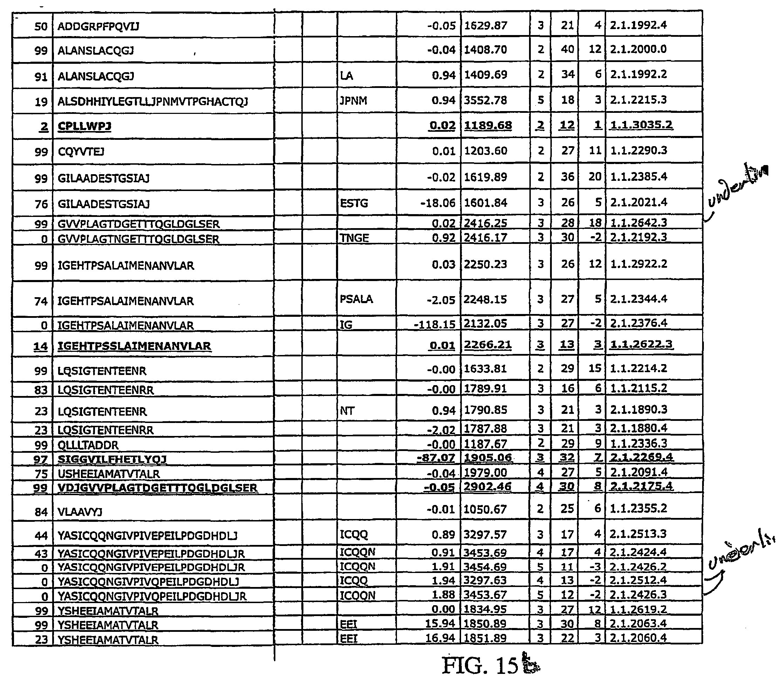

Figure 15 shows an embodiment of the present teachings that relates protein group information to the user.
Figure 16 shows an embodiment of the present teachings that permits interaction with the report in order to visualize inter group relationships.
Figure 17 is a block diagram that illustrates a computer system upon which embodiments of the present teachings can be implemented.

The section headings used herein are for organizational purposes only and are not to be construed as limiting the subject matter described in any way. While the present teachings are described in conjunction with various embodiments, it is not intended that the present teachings be limited to such embodiments. Aspects of the present teachings may be further understood in light of the examples contained herein, which should not be construed as limiting the scope of the present teachings in any way.

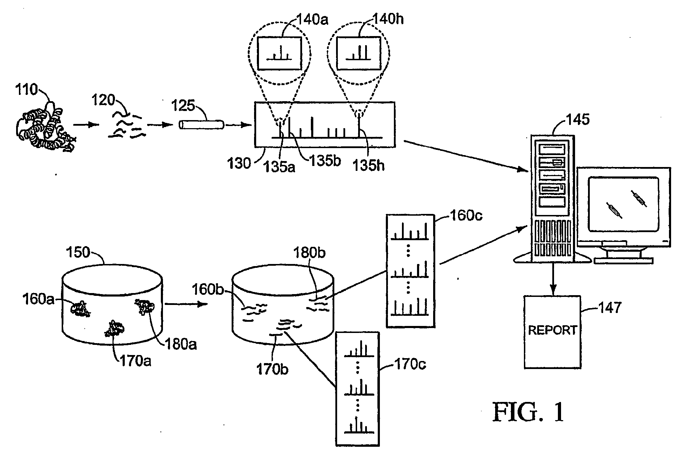

Proteins are commonly identified by comparing experimental mass spectra to theoretical mass spectra derived from a database of proteins. This process is illustrated in Figure 1. Here the protein to be identified is illustrated at 110. Between stages 110 and 120, the protein is digested with an enzyme. Typically trypsin is used as its cutting frequency results in fragment sizes well suited for mass spectrometers. The fragments at 120 are then injected in a mass spectrometer (125) that measures the mass and intensity of the peptides and outputs a mass, spectrum (130.) This MS scan identifies the masses of the various peptides. Masses are indicated by peaks in the MS scan which are illustrated.at 135a, 135b, ...135h.

Subsequent scans are typically made in MS/MS mode. This mode uses a first analyzer to select one of the peptides. The peptide is then fragmented and typically breaks along the peptide's backbone. This can result in a series of b-and y-ion fragments whose masses can be measured by a second analyzer. Several such MS/MS scans are illustrated at 140a and 140h where it can be seen with which peaks in the original MS scan the MS/MS scans are associated. This process results in a series of MS/MS spectra corresponding to the various peptides that constitute the original protein.

Typically, the next step is protein identification via database searching. This can be effected by first taking a database (150) of proteins (160a, 170a, 180a) and, using the digestion rules of the enzyme used to cut the original protein, forming in silico, a theoretical collection of peptides for each of the proteins in the database. Several such collections are illustrated at 160b, 170b, and 180b. Since the mass of each database peptide can be calculated, protein identification typically proceeds by using the mass of a precursor, such as 135b, to identify one or more possible database peptides. These database peptides can then be theoretically fragmented in a computer (145) by considering breaks along their backbones. Such fragmentation results in a series of theoretical b- and y-ions. The masses of these ions can then be matched to the masses in the experimental MS/MS spectrum in a computer (145) and the peptides matching most closely are reported to the user. Identification of the original protein can be effected by performing several analyses on the precursor ions identified in the MS spectrum and reporting the proteins (147) giving rise to the most peptide matches.

### Nature of the data

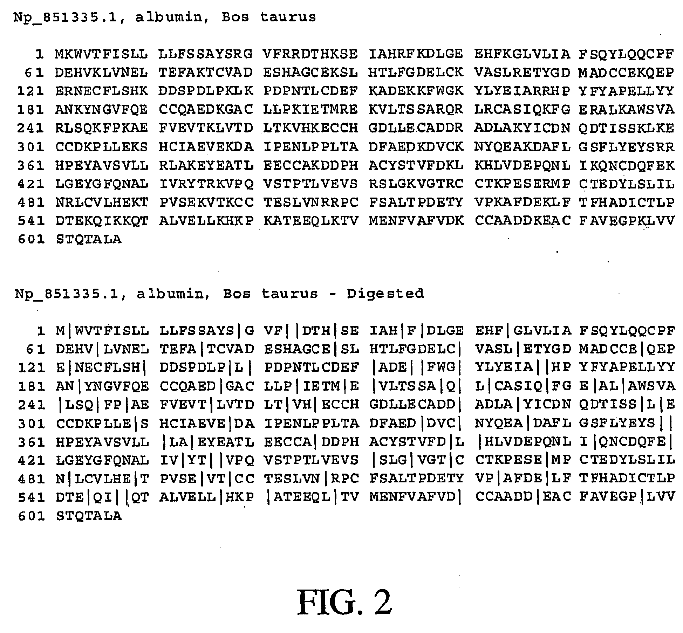

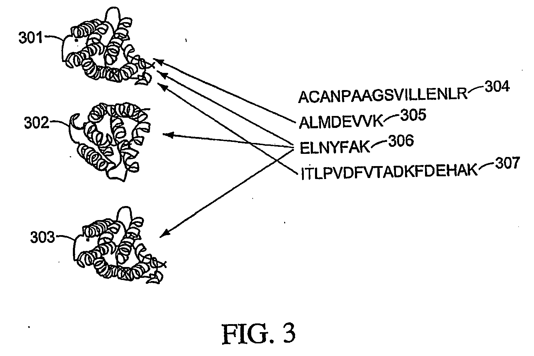

The ideal experiment involves clean data where, only one protein is present, there is no sample contamination, complete digestion occurs, each precursor is individually selectable, and each precursor is completely fragmented in a predictable manner. The ideal peptide match involves complete concurrence between the masses in experimental and theoretical spectra and a one-to-one mapping from spectra to peptide. And finally, the ideal protein match involves, identification of enough peptides in the winning protein to uniquely classify it, and no presence of unexplained peptides. Such identification would also require knowledge of all proteins. One skilled in the art will appreciate these conditions rarely exist in real life. Due to many factors such as, the presence of numerous proteins in a sample, experimental noise, imperfect identification of peptides, homologous proteins, errors in the database, isoforms, splice forms and genetic variants, protein/peptide identification typically results in a list of identified proteins that contain nearly equivalent or closely related answers. For example, the list of most likely proteins might contain three glycogen phosphorylases. Manual inspection of these three entries would likely indicate that many or possibly all of the peptides associated with these similar protein entries are common among the proteins. Figure 2 illustrates how this situation can occur. Figure 2a shows the sequence of an albumin protein from *Bos Taurus* (domestic cow.) This protein is 607 amino acids in length and the sequence listing was retrieved from the NCBI protein database and is assigned the accession number Np_851335.1. Figure 2b illustrates digestion of the protein by trypsin. Tryptic digestion generally results in cuts after each lysine (K) - X and arginine (R) - X bond unless X is proline (P). In the figure, lysine and arginine amino acids not followed by a proline (P) are designated by a vertical bar. It is generally after these bars that the protein will be cut resulting in numerous peptides. An example of where a cut is inhibited by the presence of proline (P) occurs after the arginine at location 304. One skilled in the art will also appreciate that cleavages can be missed for a variety of reasons, such as a fold in the protein obscuring the cut site, and thus not permitting the enzyme access to effect the cut. Such situations can result in somewhat "unexpected" peptides. Protein identification can be effected by identifying enough of peptides to determine that a particular protein is present. However, generally, not all of a protein's peptides can be identified. Sometimes, experiments are limited, for example, by available time, or available sample such as in the case of sample eluting from a liquid chromatography column. In some cases, not all of the peptides can adequately hold the charge after ionization and thus cannot be separated effectively in the mass spectrometer. In the case where multiple proteins exist in a sample, one peptide can give rise to the possibility of several proteins being identified. This principle is illustrated in figure 3. Here peptides 304, 305, 306, and 307 are detected. While peptides 305 and 307 only support the presence of protein 301, peptide 306 can be found in all three proteins 301, 302, and 303. As well, detected peptide ,304 does not support the presence of any of the three proteins and could be present due to a variety of reasons such as noise/contamination or an alternate form. It could also indicate the presence of a protein which is not contained in the database. This situation can be further confounded if the peptides have varying levels of confidence, for example, if peptide 304 has a very low confidence, it might indicate that one of the other proteins is present. However, if peptide 304 possesses a very high confidence, it might indicate that the true protein is not in the database.

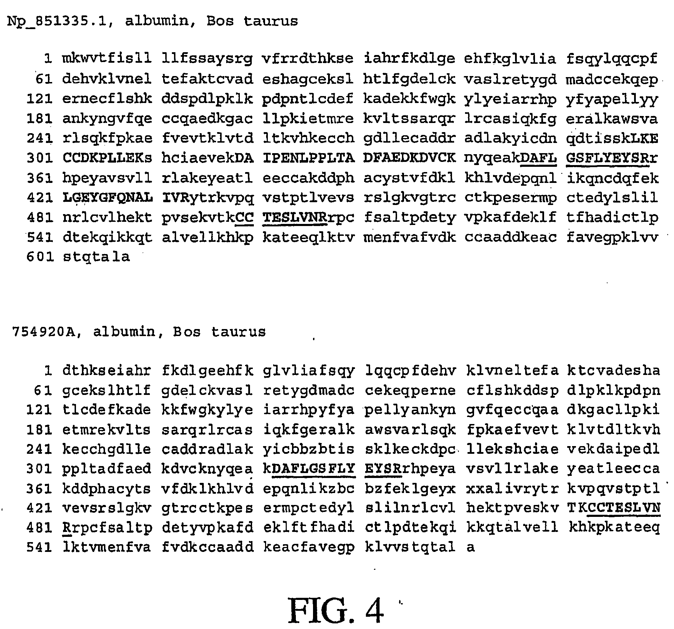

A more complex case occurs in figure 4. Here, two forms of a protein are illustrated. Figure 4a show the same albumin protein as illustrated in figure 2. Figure 4b shows an alternate form of the protein, also retrieved from NCBI with accession number 754920A. Both proteins possess the identified peptides DAFLGSFLYEYSR and CCTESLVNR which are highlighted via bold type and underlined. However, experimentally four other peptides may have also been identified; LKECCDKPLLEK, ECCDKPLLEK, DAIPENLPPLTADFAEDKDVCK, and LGEYGFQNALIVR. These peptides are highlighted with bold type and only appear in protein Np_851335.1. If this is the case, then it may be more likely that this protein is the only one present. In some cases, though, additional evidence can be present which could indicate the presence of an alternate form or another protein altogether. The peptides that suggest the second form of albumin can be accounted for entirely by the first form, thus, there is no specific evidence suggesting the second form is also present.

### Nature of the protein-grouping problem

The present teachings provide a method of performing protein identification. Some embodiments use the belief that it is more likely than not that there is only one form of a protein in a sample. Thus, unless there is evidence for more than one form of a given protein, related proteins are grouped together and a winning protein is identified. This is more likely to lead to the ideal result where winning protein(s) in each group actually appear in the sample.

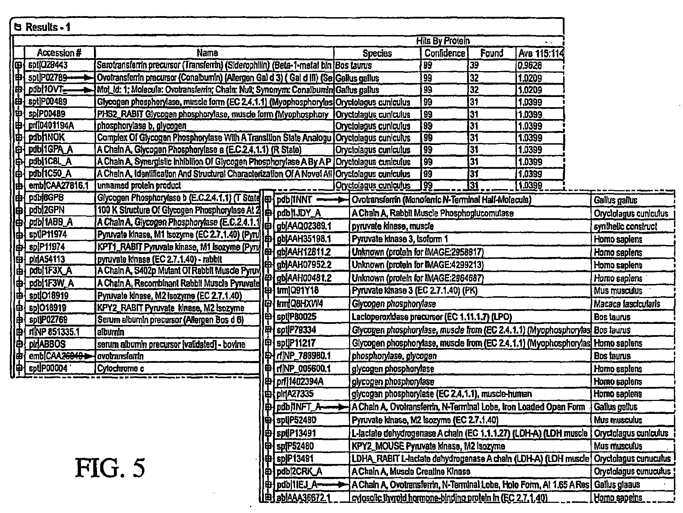

Various embodiments of the present teachings group proteins in a manner that better enables a user to determine if more than one form of a protein is present in the sample. This can be accomplished by analyzing the results of a protein database search.These results typically return a list of putative proteins, their associated peptides and associated information. The results can be organized into protein groups with each protein in a group categorized. For example, proteins can be categorized into several different types. These can include winner proteins, subset proteins, and potential alternate form proteins. Winner proteins are generally the highest scoring protein in a group. However, some situations exist where this might not be the case. For example, if the highest scoring protein in a group has already been a winner in a previous group, it can be excluded from being a winner in order to allow different hypotheses about the origin of the group to be formed. There may be one or more winner proteins in a group. Subset proteins generally have an exact subset of the peptides contained by the winner protein(s) in the group. In some embodiments, some or all of the subset proteins may be retained, particularly if there is evidence that supports their existence - for example, if they are within some margin of error of a winning protein. The user can also choose to discard some of the proteins or hide them from view based on criteria associated with the amount of evidence supporting their presence. Potential alternate form proteins generally possess a subset of peptides with the winner protein(s) in the group, but will generally also have distinct peptides of their own. Identification of these different groups and categories can provide useful information to the user. This can be important since many protein database search engines generally produce only a list of potential proteins and leave it to the user to sort out the more likely candidates. Results from such a program are illustrated in figure five. Figure 5 shows an embodiment of a typical protein identification results table. Here six different forms of ovotransferrins have been identified (see arrows) when likely only one is contained in the sample. It is probable that these results should be grouped together, and based on some form of likelihood measurement, a winner designated. However, since there could be more than one form, a means of determining if an alternate form is likely present is required. The present teachings present such methods and can allow the user to control the level of confidence required before suggesting that multiple related proteins are present. This can permit a user to dictate how aggressive the identification should be at the possible expense of including more false positives.

Various embodiments of the present teachings use an evidence-based approach to group proteins, determine their classification and identify the most likely solution. Figure six illustrates an embodiment of the present teachings. At 610, protein identification on mass spectrometer (605) data is performed. This can produce a listing of putative proteins and the peptides associated with them. This information can be stored in a database, 620. Protein grouping, 630, can be performed subsequent to protein identification results storage although there is nothing that requires the protein grouping to wait until all results are collected. In some embodiments, the protein grouping can occur as results are collected as indicated by the dataflow between 630 and 600. This can allow the grouping results to modify the data collection process. This can be useful, for example, where peptide evidence points to several proteins. In limiting the range of possibilities for proteins via the grouping process, mass spectrometer settings can be adjusted in order to look for specific peptides during subsequent data collections in order to disambiguate the results. Results can be reported in a variety of fashions (640), such as printed reports, interactive visual displays, and via database storage and recall. One skilled in the art will appreciate that there are a plurality of systems that can make use of the present teachings. For example, data can be transferred from 620 to 630 over a data connection channel such as a computer network. Once grouping is complete, reporting at 640 can occur via a data browser or sent back to the user as a computer file.

### Scores

Various embodiments utilize peptide confidence values to determine the likelihood of a protein's presence. For example, many mass spectrometry systems express the confidence of an identified peptide being present as a percentage or a P-value. These values can be combined to give a score for a protein. For example, a Total Protein Score (TPS) can be defined as the sum of the negative logarithms of one minus the individual peptide confidence values divided by 100. For sake of convenience this is referred to as the Sum of the Negative Logarithms (SNL) approach. This can be considered to be a computation of the chance that the protein is correct transformed into a form that can be easier to read. One skilled in the art will appreciate that there are many different methods of manipulating peptide confidence values or similar measures in order to obtain a score for the protein. For example, the confidences can simply be multiplied together. However the SNL approach defined herein allows the score to vary over a wider range and be more readily understood than if the confidences were simply multiplied. For example, multiplying the confidence values of five peptides with confidence values of ninety-nine percent results in a score of 0.9510 whereas the SNL approach results in a score of 10. If there are four ninety-nine percent confidence peptides, multiplication results in a score of 0.9606 whereas the SNL approach produces a score of 8. If there are three ninety-nine percent confidence peptides, multiplication results in a score of 0.9703 whereas the SNL approach produces a score of 6.

In addition to the TPS, various embodiments also compute an Unused Protein Score (UPS.) For ease of comparison, this computation can have the same basis as for the TPS. The UPS considers one protein to be the primary or reference protein and assigns a score to the secondary protein based on the peptides that the secondary protein possesses that the primary does not. The UPS of a protein relative to itself is simply the TPS.

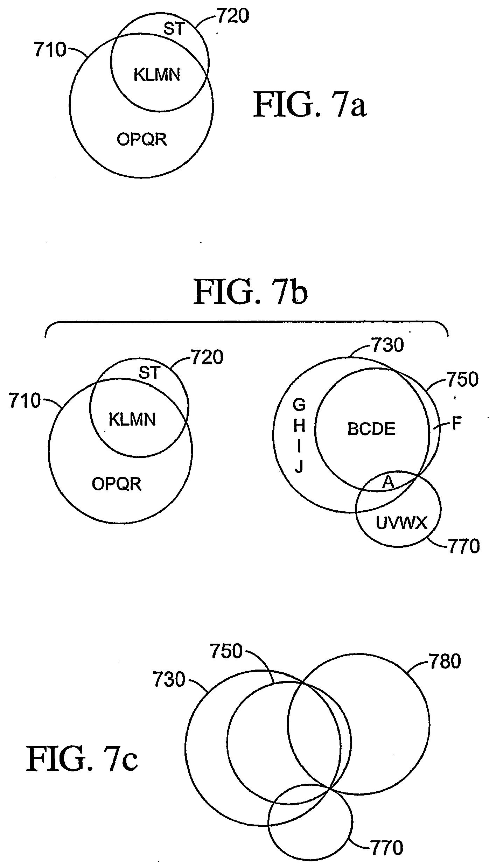

Various embodiments employ a set membership approach to perform protein grouping and calculate protein scores. For example, figure 7a illustrates that peptides K, L, M, N, O, P, Q, and R can be associated with protein 710 whereas peptides K, L, M, N, S, and T can be associated with protein 720. Thus two different protein groups can be formed. One group will contain protein 710 and 720 and will have 710 designated as the winner, another group will have proteins 710 and 720 with protein 720 designated as the winner. Some embodiments take into account the confidence values associated with the peptides so that scores reflecting the likelihood of the protein listed as the winner of being present can be computed. For example, if the confidence values associated with peptide S and T are low, then the user can infer that protein 720 is not present in the sample. Similarly, if the confidence values associated with peptides S and T are above a threshold, they may suggest that protein 720 is present.

By way of example, assume that the peptides K, L, M, N, O, P Q, R, S, and T in figure 7a have confidence values 99, 99, 83, 54, 90, 90, 82, 90, 36, and 54 percent respectively. Then, the TPS for 710 using the SNL approach is 8.8515, the TPS for 720 is 5.6378, the UPS for 720 relative to 710 is 0.5310, and the UPS for 710 relative to 720 is 3.7447. However, if the confidence value for peptide T is 15%, the UPS for 720 relative to 710 becomes 0.2644. The user can optionally set a Protein Group Threshold (PGT) that determines if a protein will be presented as the winner of its own group, implying it may be present in the sample. For the instance just discussed, if the threshold is set at 1.00, 720 might be included in the group with 710 but it would not be presented as a winner of its own group. It lacks sufficient distinct evidence, having only 0.5310 SNL units distinct of 710 (about 71% confidence). Protein 710, on the other hand, easily exceeds the threshold with both its TPS and UPS. Some embodiments use the PTG after grouping all proteins only to filter which proteins are displayed to the user. Similar to the USP, a shared protein score (SPS) can be calculated which assigns a score to the secondary protein based on the peptides that the secondary protein shares with the primary protein.

### One peptide per spectrum

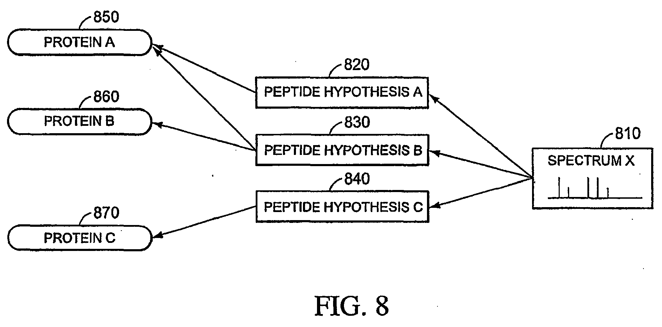

Various embodiments recognize that there can be multiple peptide hypotheses for the identity of the molecule giving rise to a spectrum. This is illustrated in figure 8. Here the spectrum 810 leads to possibility of peptides 820, 830, and 840 being present. These peptide hypotheses can have different confidence values associated with them. For example the confidence values for peptide hypotheses 820, 830, and 840 could be 99%, 67%, and 40% respectively. Thus without additional supporting evidence it is most likely that a protein containing the most probable peptide hypothesis is correct. In this case, only one protein has peptide hypothesis 820 - protein 850. Without additional information, this is often the most reasonable interpretation. Should additional evidence favor protein 860 or 870 such that they rank ahead of protein 850, some embodiments may attribute one of the lower confidence peptide hypotheses as the preferred explanation for spectrum 810. Some embodiments will assign the spectrum giving rise to that peptide hypothesis to the selected peptide and that peptide will "consume" the spectrum. This will allow the peptide's confidence value to only contribute to the score for the selected protein. While the other peptide hypotheses are still allowed to suggest the presence of other proteins, those peptide hypotheses will not be allowed to contribute to any subsequent protein scores because the spectrum that gives rise to those hypotheses has been consumed.
Conditions that can result in one peptide hypothesis being chosen over another include identification of highly likely peptides that suggest that the protein containing the putative peptide is present. For example, if several peptides suggest that a protein containing a peptide with an eighty-eight percent confidence value is present and the protein possesses an abundance of evidence leading to a high TPS, the peptide can "consume" the spectra based on the strength of the overall protein evidence. This can have the effect that a peptide resulting from the same spectra yet having a confidence value of ninety percent, but deriving from a less likely protein, may be in the same group and claim no support from this spectrum in its UPS.

Figure nine illustrates how an embodiment of the present teaching forms a protein results table (910) which can be comprised of one or more protein groups where each group can have winner proteins, subset proteins and alternate form proteins. Element 960 shows a protein group identifying the proteins in the group and giving metrics expressing the confidence that a protein is present. In this case, the group contains the TPS, the UPS and identifies the distinct spectra that contribute to the UPS. Element 920 represents the collection of proteins identified by a database search. Element 930 represents the collection of spectra used to generate peptide hypotheses. Bolded elements such as those labeled at 970 indicate spectra that have been identified as belonging to other winner proteins that are the winners of higher-ranking groups - these peptides are already 'used' or consumed before constructing this group (element 960). A link between a protein in 920 and a spectrum in 930 indicates that the spectrum leads to a peptide hypothesis that is included in the linked protein. Thus, although the spectrum S15 links to Protein 4, some embodiments will not use it as evidence to support the presence of Protein 4. The spectrum S4 links to both protein 4 and Protein 8 indicating that S4 either leads to two distinct peptide hypotheses, one contained in Protein 4 and one contained in Protein 8 or alternately, leads to a single peptide hypothesis that is contained in both proteins. If Proteins 2, 3, 5, 6, 7, and 9 each have a UPS equal to or less than 6, protein group 960 can be formed by recognizing that Protein 4 either has the highest UPS or is tied for the highest UPS and then determining all proteins that share spectra with it even if those spectra lead to multiple peptide hypotheses and/or some of those spectra have been claimed by a winning protein in another group. Because it has the highest UPS of remaining unresolved proteins, Protein 4 becomes the winner of protein group 960. Continuing, Proteins 8 and 1 share spectra with Protein 4 and will be part of group 960, even if they do not share exactly peptide hypotheses for these sequences. For simplicity sake, in this example, all spectra lead to peptide hypotheses which have 99% confidence values. In this example both the TPS and UPS are used as metrics and are expressed with the SNL scale, so the simplifying assumption that all peptide hypotheses have 99% confidence translates into an additive 2.0 units in the SNL scale for each peptide. Thus the TPS of Protein 4 is ten - 2.0 times the 5 peptides associated with it. Because Spectra 5 and 15 have been previously consumed by other winner proteins, Protein 4's UPS is 6 based on spectra 2,4, and 17 which it can claim as distinct evidence that has not been claimed by more likely proteins. Following the placement of Protein 4 as the winner of protein group 960, the UPS for the remaining unresolved proteins in group 960 are recalculated. Some embodiments would also show the protein(s) in higher ranking protein group(s) that have consumed spectra 5 and 15 common to the winner in this group, Protein 4. Continuing with the two remaining proteins in this group, the TPS of Protein 8 is. 6 due to having cited 3 spectra, while its UPS is reduced to 4 because Spectrum 4 has been consumed by Protein 4. Protein 1 has a TPS of 4 based on two spectra, but Spectrum 19 has been claimed already in a higher ranking group while Spectrum 17 has been claimed in this group by Protein 4, leaving Protein 1 with a UPS of 0. The spectra consumed by each protein are indicated in the "Spectra" column. Processing can continue by updating the UPS of all proteins, and selecting the remaining protein with the highest UPS and proceeding with the formation of the next protein group, setting this protein as the winner of this next group. Some embodiments will update the UPS of all proteins when grouping is complete so that the UPS of each protein in results table 910 reflects only the contribution of distinct spectra.

The data in figure 7b further exemplifies the way various embodiments group the proteins. In figure 7b five potential proteins (730, 710, 720, 750, and 770) have been identified with nine, eight, six, six, and five peptides respectively. In this example, all peptides are assumed to derive from different spectra, all peptides are assumed to have confidence values of 99%, and the peptides are shared among the proteins as follows. Using the SNL approach, and declaring proteins 710 and 730 as the reference proteins for each group, the following scores can be calculated.

| **Protein** | **Number of peptides** | **TPS** | **UPS** | **Peptides** |
|---|---|---|---|---|
| 730 | 9 | 17 | 17 | A B C D E G H I J |
| 710 | 8 | 16 | 16 | K L M N O P Q R |
| 750 | 6 | 12 | 2 | A B C D E F |
| 720 | 6 | 12 | 4 | K L M N S T |
| 770 | 5 | 10 | 6 | A U V W X |

Thus, the intersection between proteins 710, and 720 contains the peptides K, L, M, and N. The intersection between 730 and 750 contains peptides A, B, C, D, and E. The intersection between 750 and 770 contains only peptide A.

Some embodiments allow control of the minimal degree of intersection required for a protein to be showed as a member of a group. For example, if 3.0 SNL units of intersection were required, protein 770 would not be displayed with the protein group that 730 is the winner of as it has only 2 units of intersection with peptide A.

Some embodiments use 'competitor tolerance' to conceptually define a sphere around the winner protein of the group within which other proteins are similar enough to the winner that they may be the true protein present. This can be used to determine whether or not to show a given group of proteins that have a subset of either the winner's peptide hypotheses or a subset of the winner's spectra.

Various embodiments use a protein confidence threshold to determine the degree of distinct evidence a protein must possess in order to be declared the winner in its own group for display purposes in the result list, as already discussed in the PGT setting. Distinct evidence can be measured using a metric such as the UPS. For example, if the PGT is set to 3, protein 770 has a UPS of 6.0, and will be presented as the winner of its own group and considered present. Depending on the similarity and competitor settings, it will likely also be shown in the group having protein 730 as its winner. However, protein 750 with only 2 units UPS does not exceed this threshold PGT and would not be presented as the winner of its own group and, thus, not declared present in the sample. If the PGT is set below 2, protein 750 has enough evidence to be declared present and will be presented in the list of protein groups. Figure 7c illustrates how some embodiments of the present teachings deal with more complex data. Figure 7c contains the same proteins 730, 750, and 770 from figure 7b and adds a fourth protein, protein 780. Protein 780 however covers the previously non-intersecting evidence of 770. Thus, the unused protein score of 750 is zero and, when forming a protein group, some embodiments will not include 750 because there is no distinct evidence to support its inclusion. Some embodiments include the protein but label it in a manner so that it is apparent that it does not possess any unused evidence. The present teachings also allow for choosing to use only the highest confidence instance of each peptide rather than all the instances of the peptide. This can prevent multiple acquisitions of the same peptide contributing to several proteins' scores.

The following examples demonstrate some of the different relationships that can occur between proteins. These cases consider how various embodiments decide whether one or more proteins will be declared present in a sample. Example 1 shows the trivial case where Protein A does not share any peptides with other proteins. Example 2 shows a winner protein and another protein with only two peptides in common. This situation could indicate that Protein B is not present in the sample because there is no distinct evidence to support its presence. Example 3 demonstrates a case where two proteins share the exact same list of peptides. In this case, barring additional information such as species or other facts that can help disambiguate the two proteins, both proteins can be considered winner proteins generally with the understanding that only one of the two proteins is actually believed present in the sample.

Example 4 shows a case where Protein A has several of the same peptides as Protein B but also has an additional fairly high-confidence peptide not found in Protein B. While Protein A will be reported as present, Protein B is still shown in the group thus allowing the user to see the relationship between the two proteins. Example 5 illustrates a set of conditions somewhat similar to example four. However, the evidence for Protein B is much stronger. While Protein A will be declared the winner of the higher-ranked protein group, both proteins will be indicated as present with Protein B being presented as the winner of a lower-ranked group. Both proteins will likely be shown in the other's group to convey the relationship between then in each instance of the group. Example 6 illustrates a situation where the only evidence that would differentiate between the two proteins is in very low confidence peptides. Protein A will be considered the winner and be declared as the only protein present in the sample, because it has the higher TPS. Protein B will not be declared present because there is clearly not enough information to support two distinct forms. However, because the evidence favoring the choice of Protein A over Protein B is very weak, it is reasonable to keep Protein B in full view as a viable competitor by showing it in the group Protein A is the winner of.

**Example 1 - One protein, no shared proteins**

| Protein A | (no sharing) |
|---|---|
| LRNDGSLMYQQVPMVEIDGMJ | |
| NDGSLMYQQVPMVEIDGMJ | |
| YFPAFEJ | |

**Example 2 - Winner and uncompetitive subset protein**

| Protein A | Protein B |
|---|---|
| CCTESLVNR (99%) = | CCTESLVNR (99%) |
| DAFLGSFLYEYSR (99%) = | DAFLGSFLYEYSR (99%) |
| DAIPENLPPLTADFAEDJDVCJ (99%) | |
| ECCDJPLLEJ (99%) | |
| LGEYGFQNAILVR (99%) | |
| LJECCDJPLLEJ (93%) | |

**Example 3 - Two equivalent proteins**

| Protein A | Protein B |
|---|---|
| EEIFGPVQQIMJ (97%) = | EEIFGPVQQIMJ (97%) |
| ELGEYGFHEYYEVJ (99%) = | ELGEYGFHEYYEVJ (99%) |
| ILDLIESGJ (97%) = | ILDLIESGJ (97%) |
| ILDLIESGJJ (9%) = | ILDLIESGJJ (9%) |
| JFPVFNPATEEJ (99%) = | JFPVFNPATEEJ (99%) |
| LADLIER (5%) = | LADLIER (5%) |
| LCEVEEGDJEDVDJ (99%) = | LCEVEEGDJEDVDJ (99%) |
| QAFQIGSPWR (99%) = | QAFQIGSPWR (99%) |

**Example 4 - Competitive subset protein**

| Protein A | Protein B |
|---|---|
| AVCVLJ (81%) | (not shared) |
| GDGPVQGTIHFEAJ (99%) = | GDGPVQGTIHFEAJ (99%) |
| LACGVIGIAJ (99%) = | LACGVIGIAJ (99%) |
| TMWHEJPDDLGR (99%) = | TMWHEJPDDLGR (99%) |

**Example 5 - Two proteins with strong evidence**

| Protein A | Protein B |
|---|---|
| AVLJDGPLTGTYR (99%) | AVLJDGPLTGTYR (99%) |
| AWQDPALJPLALVYGEATSR (99%) | (not shared) |
| (not shared) | DFPIADGER (99%) |
| EPISLSSQQMLJ (94%) | (not shared) |
| VGDANPALQJ (99%) | VGDANPALQJ (99%) |
| VLDALDSIJ (99%) | (not shared) |
| YGDFGTAAQQPDGLAWGVFLJ (80%) | YGDFGTAAQQPDGLAWGVFLJ (80%) |

**Example 6 - Second protein with weak evidence**

| Protein A | Protein B |
|---|---|
| LIFAGJ (4%) = | (not shared) |
| (not shared) | QLAQJ (1%) |
| TITLEVEPSDTIENVJ (99%) = | TITLEVEPSDTIENVJ(99%) |
| TLSDYNIQJ (99%) = | TLSDYNIQJ (99%) |

### Reduction of false positives protein identifications

The present teachings can provide a method that reduces false positive protein identification by applying domain-specific rules. For example, leucine (L) and isoleucine (I) are isomers and lysine (K) and glutamine (Q) differ only slightly in mass and can easily be mistaken for each other. Thus the two peptides AAAAIAAA, and AAAALAAA possess very similar masses and few mass spectrometers can differentiate between these peptides even via fragmentation. Various embodiments will assume that there is only one of the two peptides present and accordingly use the spectrum to support the existence of only one protein and in so doing will not use the spectrum as distinct evidence for both the protein that has the Ile-containing sequence and the protein that has the Leu-containing sequence. Similarly, the two peptides AAAAFWAAAK, and AAAAWFAAAK would require extremely high quality evidence to differentiate between them, and in the absence of evidence, only one form should be assumed present. This group of domain-specific rules are of a common type in that they address how to deal with the resolution of the identity of an observed molecule; the competing peptide hypotheses to explain the observed molecule are therefore identical or nearly identical in mass (within the variation of a single peak). An initial assumption can be that one spectrum has only one true molecular identity. Only with sufficient evidence to justify the presence of more than one molecule in a spectrum should more than one peptide identification believed per spectrum. The null hypothesis assumption will generally be that many peptide hypotheses for a spectrum derive from one molecule in the solution, therefore only one peptide hypothesis is actually correct.

Another group of domain-specific rules can recognize related but distinct identified molecules. An example of this can be found in dealing with chemical deamidation whereby amino acids containing amide moieties may be converted to their acid analog. The particular problem with this modification is that the modified amino acid is equivalent to another amino acid: deamidation of N is equal to D and Q wit deamidation is equal to E. As these pairs are fairly conservative substitutions, it is not unlikely that a database of proteins would contain two homologous proteins with N/D and Q/E variations in otherwise identical stretches of sequence. This means that a difference in these pairs of amino acids can have two distinct origins - genetic or chemical. When a D or E is present in an identified peptide, often, it cannot be determined whether the acidic form residue is the direct result of translation of the genetic sequence or deamidation of a genetically indicated amide form. In such cases, there is generally an direction dependant effect for example, N and Q can be converted to D and E, respectively, but not in the reverse direction. Issues such as these can arise via the presence or combination of several features such as a chemical modification whose net result is equivalent to another amino acid (with or without modification), a modification that occurs with reasonable enough frequency that it cannot be ignored, and two ambiguous amino acids constituting reasonably likely substitutions. This issue can present a problem to protein identification because the different amino acid sequences indicate different proteins and often there is no way to determine for two distinct observed molecules whether the true physical origin is one or two proteins: molecule one could be AAANAAA from protein one and molecule two could be AAANAAA with deamidation from the same protein or molecule one could be AAANAAA from protein one and molecule two could be AAADAAA from protein two (AAANAAA with deamidation is chemically exactly the same as AAADAAA with no modification). Only by using external factors like knowledge of the species of origin of each protein sequence in the database vs. the species actually being analyzed, the probability of the modification, the probability of the substitution, etc, can one interpretation be favored over the other. Some embodiments will treat this issue by assuming the simplest explanation, the explanation involving the declaration of fewer proteins.

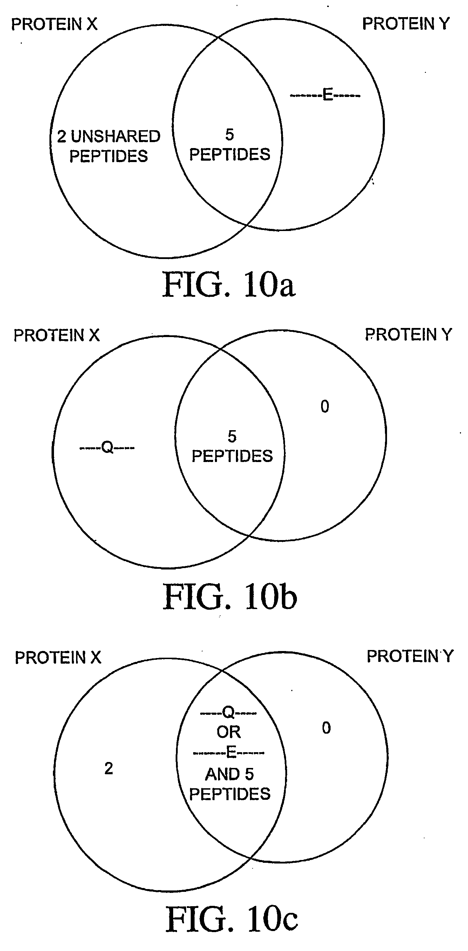

Figure ten illustrates how some embodiments group proteins when effects like deamidation are to be accounted for. In figure 10a, proteins X and Y are shown sharing five peptides, protein X has two unused peptides and protein Y has one unused peptide. However, protein Y's unused peptide is identical to an unused peptide of protein X except for a deamidation resulting in a conversion of a glutamin to glutamic acid. Since this is the only piece of additional evidence supporting the presence of protein Y, it is more likely that protein X is the only protein present and it has suffered a chemical modification. This scenario is illustrated in figure 10b where the native version of the peptide is grouped with protein X. Some embodiments report the two proteins, some will report only protein X and modify the peptide when listed, and some embodiments will only report protein X but report both the native and deamidated peptides (figure 10c). These are choices that a user can make during configuration. Such decisions can depend on contextual knowledge of the sample or other factors such as the user's degree of comfort with a given rule. One skilled in the art will appreciate that the forgoing does not limit the types of domain knowledge that can be incorporated into various embodiments and instead is intended to demonstrate how such knowledge can be used to refine the results. Some embodiments will also recognize that both related forms of a peptide may not be observed in a set of data, but the relation can be hypothesized by comparing observed peptides to the database sequences for implicated proteins. For example, if a search is conducted without allowing for deamidation as a modification, a peptide might be identified AAADAAA suggesting distinct evidence for a protein A. However, by comparison of this sequence to the sequences of other proteins that are identified in the set, it may be recognized that this molecule could also be AAANAAA with deamidation pointing to a highly confident protein. The simplest solution is the one invoking only one protein, most likely preventing one false positive protein identification.

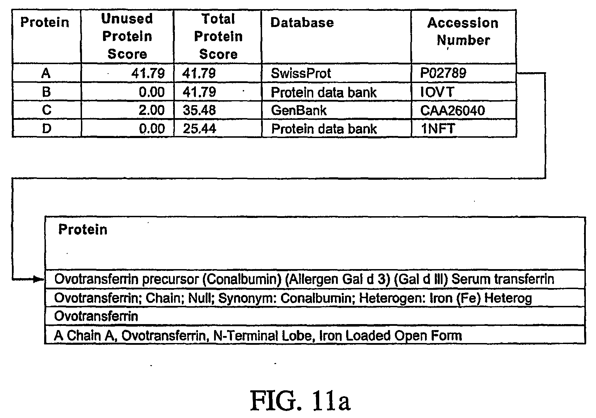

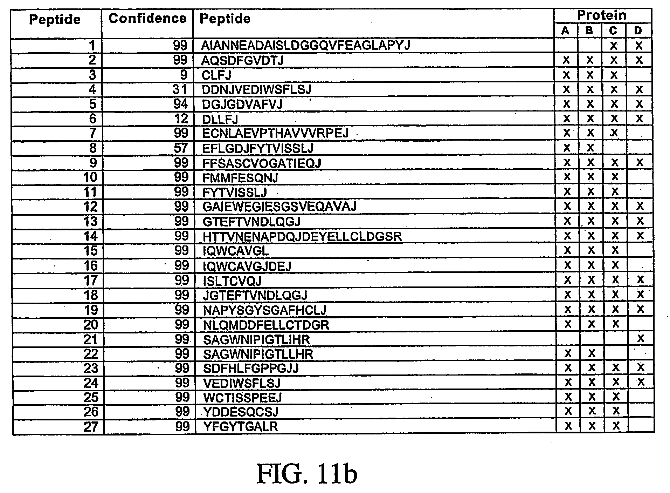

Figure 11 illustrates a group of proteins and represents the intersection in a table format instead of a Venn diagram. Illustrated is a group of proteins. The peptides identified by the mass spectrometer are contained in the column titled "Peptide" associated confidence values are contained in the column titled "Peptide Confidence." Various embodiments perform a database search to identify proteins and return a list. In this example, four proteins have been identified. These are contained in the column "Protein Name,' and their accession number is indicated in an adjacent column. The last four columns indicate to which protein each of the peptides is associated. Protein A contains 25 of the identified peptides, as does protein B. In fact, both of these proteins contain the exact same peptides and this is reflected in their Total Protein Score (column 3.) An additional metric can be the Unused Protein Score (UPS) is provided in column 2. This is the unused protein score and it relates information about the difference between two proteins. For example, protein C has only nineteen peptides, but one of them is not contained in protein A. Thus, the UPS can be computed in a similar fashion to the Protein Score except that the confidence values of the non-intersecting peptides are used in the computation. Thus, since the one non-intersecting peptide has a confidence of 0.99, the Unused Protein Score is 2.00. Protein D contains peptides mostly found in the prior three proteins but also appears to possess a unique peptide. However, the Unused peptide Score is zero. While an UPS of 2.00 could be used, in this instance, the only difference between the unique peptide and the peptide immediately below it is that one Isoleucine is a Leucine. Since these two amino acids are isomers, these two answers may be alternative hypotheses for the same spectra, and favoring the choice of the ...LHR hypothesis over the ...IHR hypothesis can result in a simpler solution at the peptide level - one fewer protein is necessary to account for these spectral data. The evidence supporting the presence of Protein D can be considered weak and thus the USP of zero. This is an example of how some embodiments build domain knowledge into the grouping problem.

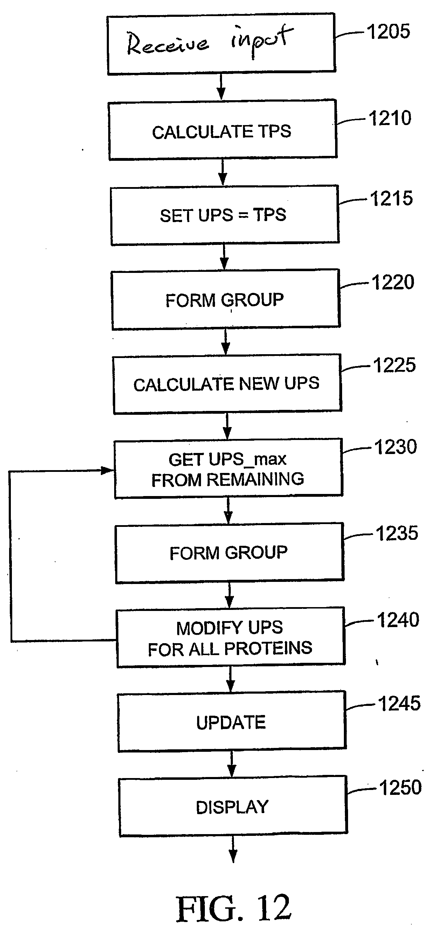

Figure 12 illustrates an embodiment of the present teachings that can be used to form protein groups. The method involves first receiving a set of input information (1205). This is typically a set of putative peptide identifications and their associated proteins returned from a protein identification search. Such searches generally operate on a set of mass spectrometer data however, one skilled in the art will appreciate that the present teachings can be used on sets of similar data that may arise from other analysis techniques such as N-terminal peptide sequencing. Associated with the peptide information is generally a confidence value or metric that can be used to infer the quality of a hypothesis to explain the observed data. This value can be related to conditions such as operating characteristics of the instrument, error models, experimental conditions, precision of database search results or other factors related to peptide identification. These confidence values can be used to calculate a Total Protein Score (1210) for each protein. A total protein score can indicate a method of assigning a quality or certainty value to a protein based on all of the evidence that supports it-in some cases without consideration of contextual relationships with other proteins. One method of calculating such a score involves the use of the cumulative probability approach discussed herein using the Sum of Negative Logarithms calculation method. Each protein can also be assigned a metric relating to the number and quality of the peptides leading to the premise that the protein is contained in the sample, not necessarily all the identified peptides pointing to the protein. This type of metric can involve analyzing relationships among proteins. An embodiment of this metric has herein been referred to as the Unused Protein Score. Because no protein groups have yet been formed, no spectra have been used so the UPS for a protein can be set to the protein's TPS (1215.) As a starting point, a first protein group can be formed at 1220 by locating the protein with the highest UPS and designating this the winner protein for the first group. If there are multiple proteins with the same score and peptide set, they can all be designated equivalent winners for the group. This can occur with the understanding that only one of the winners is likely present and can be identified as such in the absence of additional evidence. Other members of the protein group can be found by identifying all proteins that share peptides with the winner protein (s) and calculating Unused Protein Scores for them relative to the winner protein (s). Peptides that are included in calculating Unused Protein Scores are generally peptides whose originating data, (in the case of mass spectrometry, a mass spectrum) has not been used by a peptide to identify a winner protein. This recognizes that a single piece of originating data can lead to multiple peptide hypotheses. However, despite multiple peptide hypothesis, some embodiments use the assumption that only one molecule can be identified per spectrum unless evidence shows otherwise. When a piece of previously unused originating data is used to support the presence of a winner protein, the piece of data is said to be consumed. Some embodiments will assess whether there is evidence to support the presence of more than one physical molecule being analyzed in a piece of data like an MSMS spectrum. If this is shown to be justified, then these embodiments would allow a spectrum to be used as distinct evidence in support of more than one protein. This can lead to the situation where the spectrum might not be consumed by the first winner protein that cites it. The information associated with the winner proteins, subset proteins, and potential alternate form proteins can be stored for later use. At 1225 the UPS values of all proteins are updated using only peptides in the calculation that have originating data that has not been consumed by the winner of this first group. If further grouping is desired, the protein with the highest UPS that has not yet been declared a winner protein of a group can be used to start another group (1230), where the group is formed at 1235 by essentially repeating the steps used in forming the group at 1220. The arrow from 1240 to 1230 indicates that the process can continue until the user desires to stop forming groups. The process can be stopped automatically when the confidence value of the last group formed is below a prescribed cutoff confidence for display or storage, or the list of proteins has been fully exhausted by rationalizing each protein in the full set as either declared a winner protein of a group or a subordinate protein to winner protein (subset protein or potential alternate form with insufficient distinct evidence. Because the act of forming each additional group can alter the used/unused status of peptides cited by subordinate proteins listed in higher-ranking groups, the UPS for all subordinate proteins in all groups can be updated to reflect the final state at the end of the group forming process at 1245. Updating all UPS scores can involve recalculating the UPS for all proteins based on the final set of winner proteins declared in the set and the evidence and peptides they claim. Grouping resulting can be stored or displayed at 1250 and can be of many forms such as results files, HTML pages, other computer representations and printed reports. Various embodiments use visualization controls to determine the manner and which information of each protein group is stored or displayed.

In general, the term "protein group" is a set of proteins that share some sequence or physical evidence. Consistent with some embodiments, the methods described herein are driven by shared physical observations. Some embodiments carry out formation of groups using sequence similarity methods alone without consulting physically observed data.

### Visual Representation

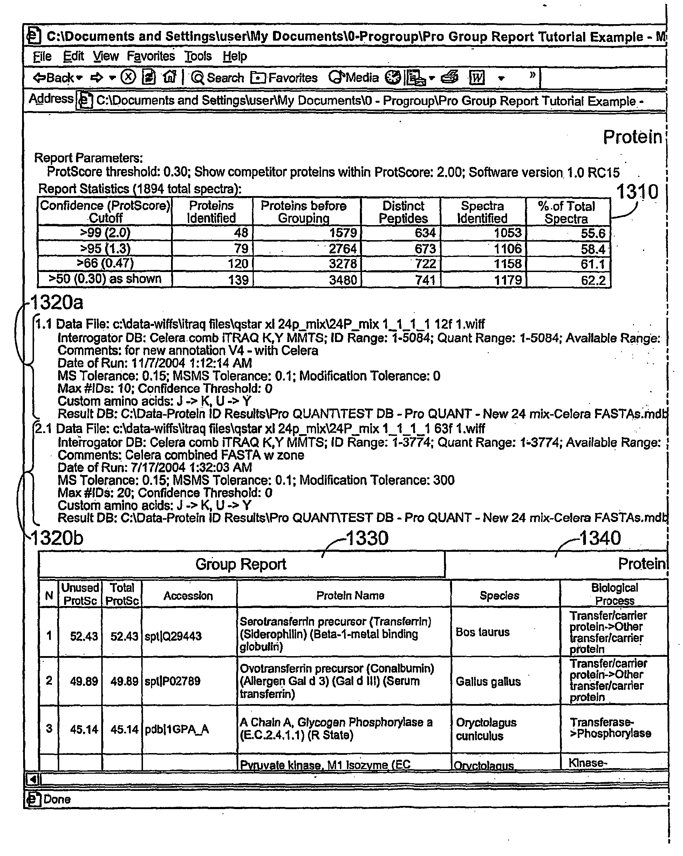

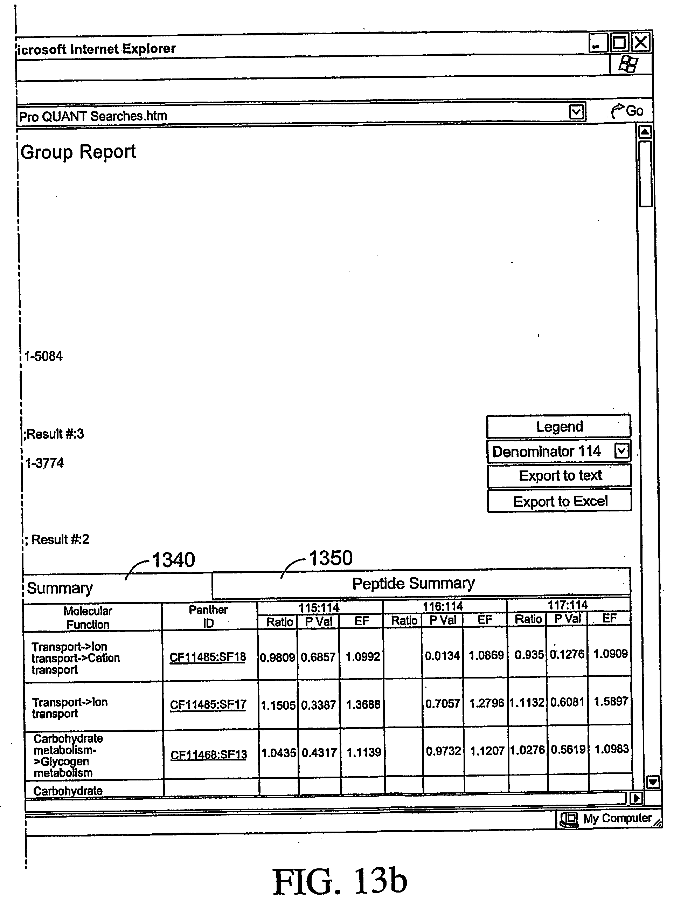

Various embodiments display protein grouping information visually using computer user interface components and principles such as spreadsheets, tabbed sheets, fontification, font styles and color coding. Figure 13 illustrates how an embodiment of the present teachings can organize the information. Information can be organized into general grouping statistics such as in table 1310, information about the search parameters used to identify proteins, a summary of the proteins identified in the tab sheet at (1340), a summary of the peptides identified in the tab sheet at 1350, and a protein group visualizer in the tab sheet at 1330.

Some embodiments convey general information about the grouping analysis. For example, table 1310 can allow the user to quickly assess how many proteins and peptides have been identified. The table gives statistics at several protein confidence thresholds, 99%, >95%, and >66%, and the last row shows statistics for the Protein Score Threshold used in the subsequent report. In this particular case, it is set to 50% confidence (Protein Score = 0.3). The table column entitled "Confidence (Protein Score) Cutoff' shows the protein confidence cutoff applied to calculate the rest of the values in that row. It is listed as both percent confidence and as its Protein Score equivalent. The table column entitled "Proteins Identified" shows the number of proteins identified at each confidence threshold. This number is a suggested minimal set of proteins based on the grouping analysis and can represent the maximal number of proteins reportable with a given level of confidence. The table column entitled "Proteins before Grouping" shows the total number of proteins in the result set that have a TPS indicating confidence over each threshold. It is the number of proteins typically reported in the absence of a grouping analysis and is information typical of many protein identification tools that do not use grouping analysis. The table column entitled "Distinct Peptides" shows the number of distinct peptides associated with the identified proteins. This statistic can contain low and high confidence peptides that are associated with proteins identified over the threshold. Various embodiments use this metric to determine how many modified variants can be found by searching with and without modifications. The column entitled "Spectra Identified" reports the total number of spectra associated with the identified protein set at each threshold. Various embodiments estimate the extent of redundant MS/MS acquisition by determining the ratio of spectra identified to distinct peptides identified. For example, the 99% confidence level in table 1310 shows 1053/634 = 1.66, indicating that on average, each distinct molecule is acquired 1.66 times. The table column entitled "% of Total Spectra" reports the percent of the total spectra in the data used in the report that are associated with a peptide associated with a protein identification. In this embodiment, the total number of spectra is reported at the top of the table, next to the "Report Statistics" title. Additional information such as that at 1320a and 1320b can tell the user details of the database searches, including any custom amino acid translations from a Data Dictionary at the time of search, database names, and where the results are located.

### Protein and Peptide Summary Information

Some embodiments show the user protein summary information on a tab sheet (1340) that lists one or more winner protein in each group in the protein group tab (1330). To facilitate examination, the proteins can be sorted in order of decreasing confidence by using the UPS as a metric. In the exemplary data, the highest confidence protein ID in group number 1 has a UPS of 52.43. Some embodiments color code the UPS column cells to assist the user in assessing the protein confidence. For example dark green can be used for proteins with a UPS greater than 99% in order to indicate that these proteins could be considered correct without validation, if one is willing to accept one error in one hundred. Similarly cells can be colored light green to show confidences between 95% and less than or equal to 99% indicating that these proteins have a good chance of being correct. Addition thresholds and color can be created as needed to define additional categories such as low confidence and most likely incorrect.

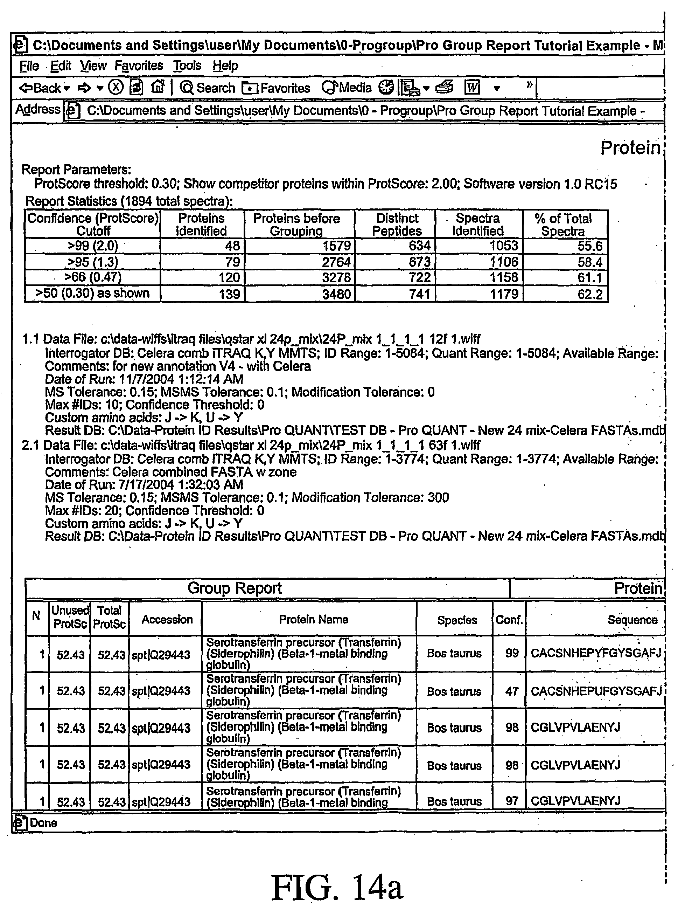

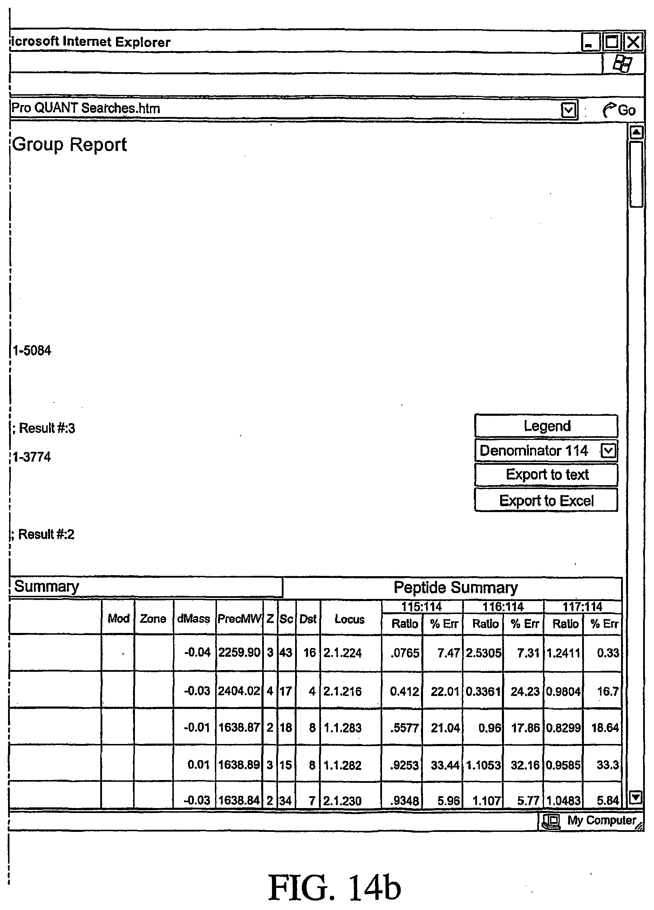

Peptide Summary information can be conveyed to the user via a peptide summary tab sheet as in figure 14. This information can contain a list of some or all of the peptide associated with the proteins listed in the Protein Summary tab sheet. Similar methods of displaying the data as used for the protein summary information can be employed. For example, the TPS and UPS for the protein with which a peptide is associated can be displayed along with the protein's name. Peptide sequence information and, and associated information such as the confidence score and any other experimental data can be included. Some embodiments permit selection of a peptide and the expansion of the table to show all proteins in which the selected peptide can be found.

### Group Information

### Visual encoding of protein group information

The present teachings include a protein group viewer that can facilitate examination of complex relationships among proteins. This viewer can take the form of a tab sheet containing the different protein groups, their associated peptides and associated parameters relating to the search and/or the data collection process itself. An embodiment of the present teachings is illustrated in figure fifteen. This example shows the thirteenth protein group in a Protein Group Report. The group can be divided into two sections: the protein section on the left and the peptide section on the right. Functionality can be provided to expand or collapse a protein group. The protein group in figure fifteen is expanded so that the group's proteins and associated peptides can be viewed.

Formatting to denote relationships with respect to the winner protein(s)' being declared in an instance of a protein group can be performed. Relational information can be encoded using visual differences such different fonts, colors, shading, and/or patterns. Broad formatting rules can be defined to help differentiate categories of proteins. For example, any protein that is declared present somewhere in the list can be shown in normal text, while italics can be used to list proteins that are believed not present via some logic - for example, they may have a subset of the peptides possessed by some other protein. A protein believed to be present in the protein group can be indicated by a non-italicized typeface. As well, underlining can be used to indicate proteins that have peptide sequences in addition to the peptide sequences in the winner protein(s), where as proteins that have an equal set or subset of the peptides contained by the winner can be indicated by an absence of underlining. These different rules can be combined to label and convey information about the relationships. Several examples follow.

A winner protein believed to be present can be indicated by a bold typeface - in figure fifteen there are several equivalent winners, they are all in bold as they share the same peptide set. Subset proteins, proteins with an exact subset of the peptides contained by the winner protein(s) in the group, can be shown by formatting their name so that they and non-bold, italicized, and non-underlined. Proteins that have a subset of peptides with regard to the winner protein(s) and possess additional peptide evidence where the evidence is consumed by winner proteins in other groups can be indicated by being italicized, non-bold, and underlined. Proteins that have a subset of peptides with regard to the winner protein(s) and possess additional peptide evidence where the evidence is not consumed by winner proteins in other groups can be indicated by being bold, non-italicized and underlined.

A protein group can be presented with respect to the protein being declared the winner in that instance of the group. For example, if two related forms of a protein are declared present in the list (ie. sample) - one with very high confidence and the second with confidence just over a pre-defined threshold, the first time the group is shown, formatting features can be used to present the high confidence primary form. All relationships between the proteins and peptides in the group can be shown with respect to the primary form. The second time the group is shown, the much lower confidence secondary form protein can be presented as present, and all the formatting altered to show relationships among proteins and peptides in the group with respect to this protein. The appropriate metrics such as the TPS , UPS, and other parameters can be included for each protein.

With regard to the peptides, relational information can also be coded using visual methods. For example, in figure fifteen, information is coded as follows. Peptide sequences that are contained by the winner protein in an instance of a protein group can be shown in a non-bold, and non-underlined font. In order to show peptide sequences that are not contained by the winner and consume spectra that are not used by the winner protein(s), a bold, underlined font can be used. Peptides that are not contained in the winner protein(s) but whose spectra have been consumed by proteins in another group can be indicated by non-bold, underlined font. The appropriate metrics such as the confidence value, other search parameters are included for each peptide.

Such distinctions, can allow the user to see which peptide identifications provide strong evidence to suggest the presence of additional protein forms in the protein group. One skilled in the art will appreciate that other relationships and formatting conventions, can be used without altering the nature of the present teachings.

One skilled in the art will appreciate that many methods can be designed in which the displayed or stored content of groups can be controlled differently than the full protein grouping data. For example, protein groups might be displayed only if the confidence of the winner of each group is over some threshold, related proteins within each group might only be displayed if they are sufficiently similar to the winner of a group, exact subset proteins of the winner might only be displayed if they are within some margin of error of the winner of the group such that there is some chance that they the correct answer instead of the reported winner, etc. Or, by setting a Minimum Group TPS, no group with a winner protein with less than this setting will be reported. This can be considered a protein confidence cutoff. Some embodiments also provide a separate setting- Minimum Confidence for Multiple Forms - to control the reporting of multiple forms of related proteins. For example, if this parameter is set to 95%, at least a combined 95% confidence worth of non-intersecting peptide (UPS) evidence is required before two proteins with some shared peptides can both be reported as winner proteins and appear as such in two separate protein groups. For example if two splice variant proteins each have one peptide that is not shared, the protein with the non-intersecting peptide of higher confidence can be reported as the winner of a protein group. If the peptide confidence of the non-intersecting peptide (source of non-zero UPS) from the lower confidence splice variant protein is greater than the minimum confidence for multiple forms threshold, the second splice variant can also be reported as a winner protein in a second group. If the confidence on this peptide is less than the parameter, it will only be reported as a potential alternate form in the same group where the dominant splice form is the winner.

By setting a Show Competitors within Protein Score parameter, any subset or potential alternate form protein with a difference in protein score in SNL units of the winner protein's TPS will not be shown in the results. Some embodiments make specific exceptions to this parameter to allow proteins to be displayed in a group if they have any non-zero UPS or UPS over some specified level, thus indicating they are potentially present as an alternate form.

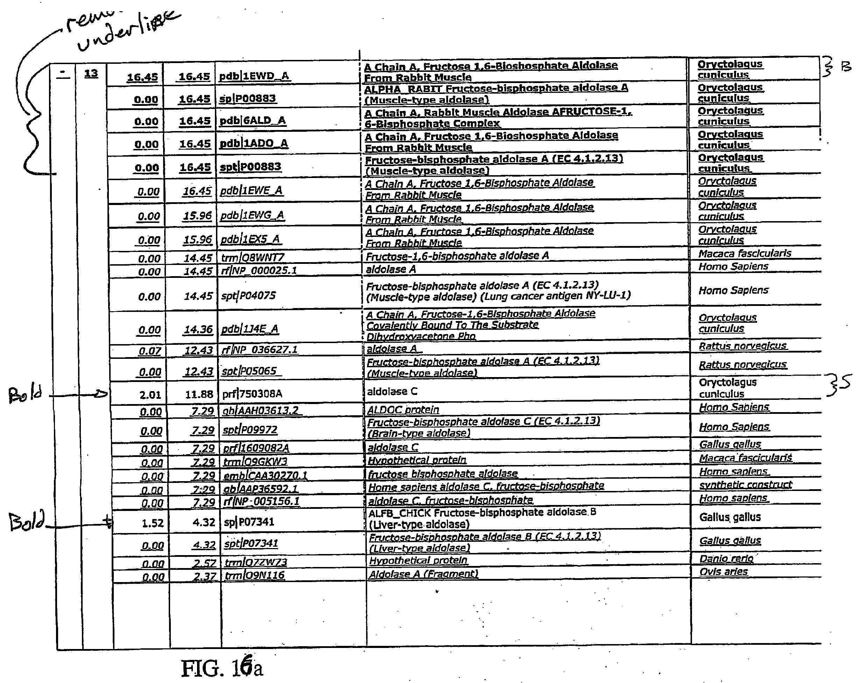

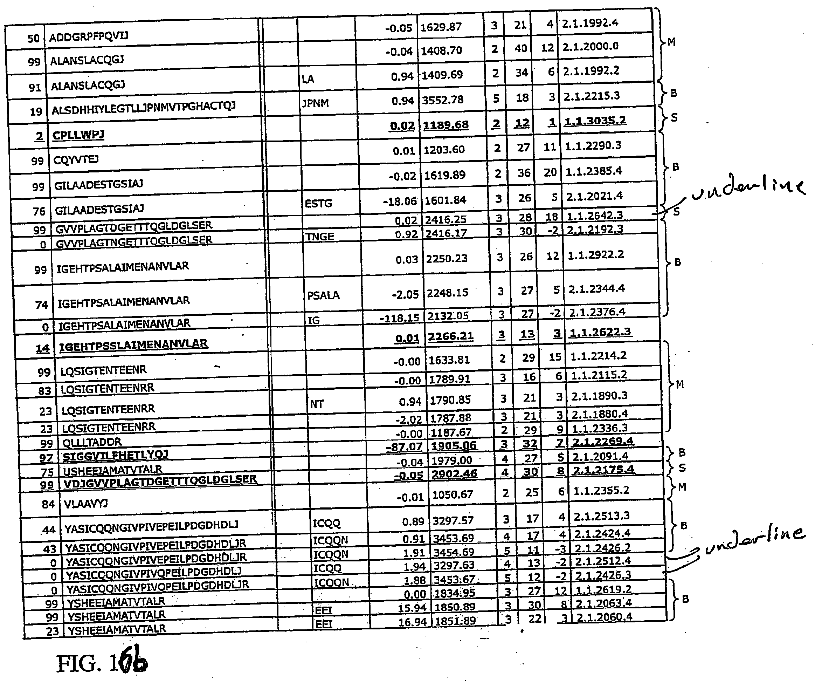

The present teachings can provide interactive data analysis methods that permit examination of containment relationships among proteins and peptides within a protein group. For example, selecting a protein in a protein group can shade the selected protein and all peptides in the protein group that it contains. Thus, selecting a winner protein will reveal that many, perhaps even all of the peptides in the group are associated with the selected protein. Selecting a subset protein would reveal that some, but not all of the peptides contained by the winner protein(s) are also contained by selected subset protein. Similarly, selecting a potential alternate form protein will reveal that it contains at least one non-shared peptide as compared to the winner protein(s). Various embodiments permit the selection of a peptide in a protein group and will indicate by a change in color, pattern, or some other method in the cell of the selected peptide and the cells of all proteins in the group that the peptide belongs to. The present teachings also allow the user to examine the peptide union and disjoint sets between two proteins. For example, various embodiments allow concurrent selection of a first and second protein. When the first protein is selected the cell associated with the first protein and the cells of peptides in the protein group associated with the first protein are colored a first color. When a second protein is selected, the cell associated with the second protein and the cells of peptides in the protein group associated with second protein are colored a second color. Any peptide cells that are common to the two selected proteins will be colored a third color. Figure 16 illustrates an embodiment of the present teachings where the uses three colors to demonstrate this principle. The blue cells, as indicated by the letter B on the right hand side corresponds to the first protein, salmon colored cells, as indicated by the letter S on the right hand side of the cells, corresponds to the second protein, and the magenta cells, as indicated by the letter M on the right hand side of the cells, corresponds to the shared peptides.

### Protein Grouping Application to Quantitation Analysis

### Protein Form-Specific Quantitation

Protein identification analysis is often done in conjunction with quantitative analysis to determine both absolute and relative quantitative measures for peptides, proteins, and features such as modifications. Quantitative analysis can be achieved a variety of ways such as direct quantitation measurements via peak integration, methods using internal and external quantitation standards, and reagent-based methods using reagents such as the ICAT Reagents and the iTRAQ Reagents (both from Applied Biosystems.) Regardless of method, error in protein identification can propagate to the various types of quantitative analyses. For example, a general approach to determine the differential expression of proteins in a sample between two states of interest is to digest the proteins and identify peptides and also determine a ratio of the intensity of each peptide in one state vs. the other. In some cases, the proteins present in the sample can be determined by assembling evidence from identified peptides as described by various embodiments herein and then the differential expression ratio of each protein between the two states can be determined via methods such as statistical averaging of the ratios for each of the peptides used to identify it. If all peptides uniquely indicate one protein, this process can be simple. However, if there are multiple related forms of proteins identified in a set where some peptides, or at least spectral evidence, may be common among more than one protein, the quantitation accuracy of each form of the related proteins present can be enhanced using protein grouping methods such as those described herein. For example, if a protein group shows a dominant protein isoform with eight peptides and some evidence for a second isoform based on one distinct peptide with six peptides in common with the dominant isoform, a grouping and protein confidence analysis concluding that both forms are present would dictate that the protein quantitation for the dominant form should be based on only the two distinct peptides indicating this form and the protein quantitation for the second form should be based on only the one peptide that is distinct to it with respect to the dominant form. The six peptides that are common to the two forms might not be useful to express the quantitative difference between protein forms. If however the grouping and protein confidence analysis concludes that the one distinct peptide for the secondary protein form is too low in confidence to reasonably support the declaration of two isoforms, the protein quantitation of the singly declared isoform would then be based on the quantitation of all eight of its peptides. Resolution of protein groups can result in more accurate protein quantitation. Some embodiments will automatically determine protein form-specific quantitative analysis following protein identification.

### Differential Modification and Form-Specific Quantitation

Complications in protein form-specific quantitation analysis can include the possibility of the fractional occupancy of modified sites on identified peptides. An example or this arises in the case of a protein that has three observed peptides where two of them are related as phosphorylated and non-phosphorylated variants of the same sequence. If the true physical changes that occur between two states are a concomitant two-fold down regulation of the protein and an increase in the occupancy of the phosphorylation site from 10% to 40%, the three peptides for this protein will all indicate different ratios. The peptide that only exists in one state will indicate the true change in protein expression, a ratio of 0.5 (defining the ratio as (State 2 : State 1). The other two peptides can interconvert via addition or loss of the phosphate group. The observed ratio for the unmodified state of this peptide will then be the product of its change in intensity due to loss by conversion to the phosphate form and the change due to loss of protein concentration: (60% / 90%)*(0.5) = 0.333. Similarly, the observed ratio for the modified state of the same sequence will be the product of the change in intensity due to increase phosphate form and the change due to loss of protein concentration: (40% / 10%)*(0.5) = 2. This example protein then has peptides with ratios of 0.5,0.333, and 2.0, yielding an apparent change in the protein of 0.944 via an average of these three. This number may not accurately reflect the true changes in the protein or the modification occupancy. Some embodiments use a combination of any or all of the protein grouping and confidence analyses described herein, analysis for potential concomitant changes in modification of some of the peptides for a protein, and efforts to observe additional modified states of peptide sequences that would support or discredit hypotheses of concomitant differential modification and differential protein expression. Some embodiments use domain analysis as a mechanism to hypothesize sequences that may have unobserved modified states, allowing these states to be indentified. For example, if a protein has six peptides that are highly consistent in the ratio they indicate but one peptide that indicates a completely different ratio, one possible hypothesis to explain this apparent outlier is that there is another modified state present in the sample for this seventh peptide. Knowledge of the relative frequency of modifications, particularly with respect to their reactivity or specificity toward the subject sequence can permit a targeted search for the missing states.

### Protein Grouping, Protein Identification Confidence, and Applications

### Soft Decisions in Protein Identification and Quantitation

Some embodiments approach protein identification and quantification whereby "soft decisions" are made throughout the process of evidence assembly. This can be effected by assigning certainty or quality values to any observation that can then be propagated into other levels of evidence. By contrast, a process that makes "hard decisions" makes discrete decisions or classifications in assembling and interpreting observations. For example, a set of ten peptide identifications with varying confidence levels can be assembled into a set of proteins by setting a threshold peptide confidence level above which peptide identifications will be declared correct and below which they will be declared wrong or ignored. The protein set can be determined assembling the peptides into a minimal set of proteins. This can be accomplished by identifying the smallest number of proteins that account for all the accepted peptides. An example of this arises when three peptides, A, B, and C in the set of ten have confidence values of 80%, 96%, and 99%, respectively, where A and B belong to protein one, and B and C belong to protein two. If a confidence threshold is set to believe peptides 96% confidence or better and reject peptides under this threshold, peptides B and C will be considered correct, and the minimal protein set to account for these peptides will include only protein two. Alternately, if the peptide threshold is set below 80%, all three peptides will be members of the accepted set of peptides, and both proteins one and two will be indicated as present. Based on a hard threshold, this approach makes hard decisions about the presence or absence of proteins. Consistent with embodiments described herein, soft decision approaches can be applied to the same example. For example, these two proteins can be identified as a protein group and the null hypothesis is can be formed that only one of the two proteins actually present. The total protein confidence using the cumulative probability method is 99.2% and 99.96% for protein one and two respectively. This can be calculated by the product of the chance each identification is wrong, yielding the chance that neither peptide is correct. For example, 80% and 96% for protein one have 0.20 and 0.04 fractional chance of being wrong, giving 0.20*0.04=0.008, which translates to 99.2% chance at least one of the peptides for the protein is correct. Because protein two has higher confidence, protein two is most likely the protein present.

The presence of a second protein in the sample, protein one, may then depend on the presence or absence of peptide A. Thus, the confidence that there is a second form present can be calculated at 80%. The specified peptide thresholds in the hard decision method correspond directly to the distinct protein confidences in the soft method: peptide confidence thresholds set over 99 yield zero proteins, over 96 yield one protein, and below 80 yield two proteins, while the soft approach yields the same numbers of proteins at the equivalent protein confidence thresholds. In this trivial example, the two approaches may be the same. However, as soon as there is more than one peptide in the non-intersection regions of the Venn diagram, the two methods are not equivalent. If a peptide with 70% confidence, belonging to protein one is added to the previous example, the distinct evidence in support of the presence of protein one in addition to protein two is based on two peptides with 80 and 70% confidence, which yields a cumulative distinct confidence of 94% (from 0.20*0.30=0.06 - the chance both these peptides are wrong). The approach making a hard decision at the peptide level concludes the same results - 0 proteins over 99%, 1 over 96%, and two under 80% peptide threshold. The soft decision approach with thresholding only at the end of the process at the protein level concludes 0 proteins over 99%, one protein over 96%, and two proteins below 94%. Relative to the hard decision approach, the soft approach is able to leverage poor quality peptide identifications to detect more proteins. Soft decision methods can be applied to protein grouping, protein confidence calculations, protein quantitation, and other similar problems.

### Soft Decisions in Subsequent Acquisition and Second Pass Methods

Soft decision techniques can also be applied to second pass search methods, whereby initial results are obtained and subsequently used to influence how additional data is acquired and/or how subsequent identification methods should be applied to the acquired data. For example, an initial database search can be conducted allowing for likely search space features such as common modifications, expected digest cleavage features, conservative substitutions, only proteins in the expected species, etc. Because the search space is limited to likely features, the search can locate high probability proteins quickly. A second pass can involve a much wider range of variations in feature space by constraining protein space, yielding a set of multiple searches that yield better results more quickly than a single analysis technique. Some methods such as those employed by Mascot (Matrix Science) allows users to check proteins in a preliminary list of identified proteins to subject these proteins to a second pass approach that looks for a wider range of features (modifications, substitutions, etc.) using only sequences of the selected proteins in searching for additional identifications. However, because only the proteins from the first pass are searched in the second pass, the set of identified proteins cannot be revised and the second pass can result in incorrect results.

Some embodiments of the present teachings retain the initial peptide hypotheses for each spectrum from the first pass such that additional passes alter the best answer for a spectrum by providing a more likely hypothesis for the identity. Hard decisions are also frequently applied to direct subsequent acquisitions of additional data. For example, using an initial set of identified proteins, masses of peptide variants of these proteins can be calculated and a mass spectrometer can be instructed to acquire fragmentation data on peaks in the MS spectra that may correspond to these predicted peptides. Application of the teachings herein can provide a more accurate description of the relative probabilities and relationships among proteins (for example, within protein groups) that can be used to ameliorate effects of hard decisions for searching and acquisition. For example, rather than selecting only the winner proteins in each group for subsequent acquisition or analysis, the Winners and proteins within some margin of error could be considered. For example, if the difference between the winner of a group and its closest competitor subset protein is only a 4% confident peptide, it is possible that the closest subset protein is really correct instead of the apparent winner in the first pass. This can be resolved with additional acquisition or identification of peptides: For example, if additional peptides can be located via acquisition or second pass identification analysis where they are specific to the highest subset protein, this can result in a revision of the conclusions for this protein group, now favoring as the winner what was a subset protein in the first pass. One the other hand, the protein that was the apparent winner in the first pass would then be viewed as unlikely to be present, only having 4% confidence worth of distinct evidence and may no longer be the best choice. Some embodiments may also conduct an analysis to identify differences in the sequences among similar proteins in an effort to focus or direct subsequent acquisition or analysis to find peptides that would identify the best protein.

### Computer system implementation

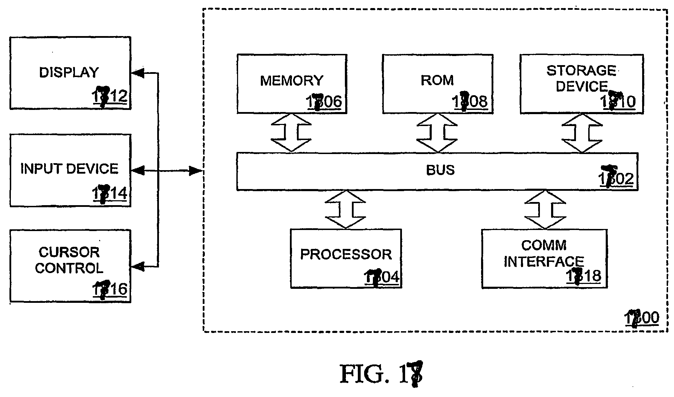

Figure 17 is a block diagram that illustrates a computer system 1700, according to certain embodiments, upon which embodiments of the present teachings may be implemented. Computer system 1700 includes a bus 1702 or other communication mechanism for communicating information, and a processor 1704 coupled with bus 1702 for processing information. Computer system 1700 also includes a memory 1706, which can be a random access memory (RAM) or other dynamic storage device, coupled to bus 1702, and instructions to be executed by processor 1704. Memory 1706 also may be used for storing temporary variables or other intermediate information during execution of instructions to be executed by processor 1704. Computer system 1700 further includes a read only memory (ROM) 1708 or other static storage device coupled to bus 1702 for storing static information and instructions for processor 1704. A storage device 1710, such as a magnetic disk or optical disk, is provided and coupled to bus 1702 for storing information and instructions.

Computer system 1700 may be coupled via bus 1702 to a display 1712, such as a cathode ray tube (CRT) or liquid crystal display (LCD), for displaying information to a computer user. An input device 1714, including alphanumeric and other keys, is coupled to bus 1702 for communicating information and command selections to processor 1704. Another type of user input device is cursor control 1716, such as a mouse, a trackball or cursor direction keys for communicating direction information and command selections to processor 1704 and for controlling cursor movement on display 1712. This input device typically has two degrees of freedom in two axes, a first axis (e.g., x) and a second axis (e.g., y), that allows the device to specify positions in a plane.

Consistent with certain embodiments of the present teachings, functions including protein, peptide and associated information input, grouping of proteins, printing, storage and presentation of results, and interactive display of results can be performed by computer system 1700 in response to processor 1704 executing one or more sequences of one or more instructions contained in memory 1706. Such instructions may be read into memory 1706 from another computer-readable medium, such as storage device 1710. Execution of the sequences of instructions contained in memory 1706 causes processor 1704 to perform the process states described herein. Alternatively hard-wired circuitry may be used in place of or in combination with software instructions to implement the invention. Thus implementations of the present teachings are not limited to any specific combination of hardware circuitry and software.

The term "computer-readable medium" as used herein refers to any media that participates in providing instructions to processor 1704 for execution. Such a medium may take many forms, including but not limited to, non-volatile media, volatile media, and transmission media. Non-volatile media includes, for example, optical or magnetic disks, such as storage device 1710. Volatile media includes dynamic memory, such as memory 1706. Transmission media includes coaxial cables, copper wire, and fiber optics, including the wires that comprise bus 1702. Transmission media can also take the form of acoustic or light waves, such as those generated during radio-wave and infra-red data communications.

Common forms of computer-readable media include, for example, a floppy disk, a flexible disk, hard disk, magnetic tape, or any other magnetic medium, a CD-ROM, any other optical medium, punch cards, papertape, any other physical medium With patterns of holes, a RAM, PROM, and EPROM, a FLASH-EPROM, any other memory chip or cartridge, a carrier wave as described hereinafter, or any other medium from which a computer can read.

Various forms of computer readable media may be involved in carrying one or more sequences of one or more instructions to processor 1704 for execution. For example, the instructions may initially be carried on magnetic disk of a remote computer. The remote computer can load the instructions into its dynamic memory and send the instructions over a telephone line using a modem. A modem local to computer system 1700 can receive the data on the telephone line and use an infra-red transmitter to convert the data to an infra-red signal. An infra-red detector coupled to bus 1702 can receive the data carried in the infra-red signal and place the data on bus 1702. Bus 1702 carries the data to memory 1706, from which processor 1704 retrieves and executes the instructions. The instructions received by memory 1706 may optionally be stored on storage device 1710 either before or after execution by processor 1704.

The foregoing description has been presented for purposes of illustration and description. It is not exhaustive and does not limit the invention to the precise form disclosed. Modifications and variations are possible in light of the above teachings or may be acquired from practice. Additionally, the described implementation includes software but the present teachings may be implemented as a combination of hardware and software or in hardware alone. The present teachings may be implemented with both object-oriented and non-object-oriented programming systems.

## Claims

1. A method of identifying proteins present in a sample, said method comprising:
(a) performing a mass spectrometry scan on said sample to produce a mass spectrum (130) identifying masses of a plurality of peptides;
(b) performing MS/MS scans on said plurality of peptides to produce a plurality of experimental MS/MS spectra;
(c) comparing the experimental MS/MS spectra to theoretical mass spectra derived from a database of proteins;
(d) providing plural peptide hypotheses for each experimental MS/MS spectrum;
(e) associating a protein score contribution to each peptide hypothesis, said protein score contribution being based on a confidence that said peptide is in said sample;
(f) determining a list of possible proteins based on said peptide hypotheses;
(g) for each protein in said list, calculating a protein score based on protein score contributions from peptide hypotheses included in said protein;
(h) identifying a winner protein based on said protein scores;
(i) consuming the experimental MS/MS spectra of peptide hypotheses included in said winner protein; and
(j) repeating steps (f) to (i), whereby repetitions of step (g) involve calculating an unused protein score for each protein in the list, said unused protein score being based on protein score contributions from peptide hypotheses present in said protein, but not including any protein score contributions from peptide hypotheses based on spectra that were previously consumed in step (i).

2. The method of claim 1, wherein the protein score contribution of a peptide hypothesis is calculated as a negative logarithm of one minus the percent likelihood that the peptide is present in the sample divided by one hundred, such that, for example, a peptide hypothesis with a 99% likelihood of being present has a protein score contribution of 2.

3. The method of claim 2, wherein calculating a protein score for each protein in the list comprises summing protein score contributions of peptide hypotheses present in said protein.

4. A system for identifying proteins present in a sample, said system comprising:
a mass spectrometer arranged to perform a mass spectrometry scan on said sample to produce a mass spectrum (130) identifying masses of a plurality of peptides and arranged to perform MS/MS scans on said plurality of peptides to produce a plurality of experimental MS/MS spectra;
a processor arranged to:
(i) compare the experimental MS/MS spectra to theoretical mass spectra derived from a database of proteins;
(ii) provide plural peptide hypotheses for each experimental MS/MS spectrum;
(iii) associate a confidence value to each peptide hypothesis;
(iv) determine a list of possible proteins based on said peptide hypotheses;
(v) for each protein in said list, calculate a protein score based on protein score contributions from peptide hypotheses included in said protein;
(vi) identify a winner protein based on said protein scores;
(vii) consume the experimental MS/MS spectra of peptide hypotheses included in said winner protein; and
(viii) repeat (iv) to (vii), whereby repetitions of step (v) involve calculating an unused protein score for each protein in the list, said unused protein score being based on protein score contributions from peptide hypotheses present in said protein, but not including any protein score contributions from peptide hypotheses based on spectra that were previously consumed in step (vii).

5. The system of claim 4, wherein the protein score contribution of a peptide hypothesis is calculated as a negative logarithm of one minus the percent likelihood that the peptide is present in the sample divided by one hundred, such that, for example, a peptide hypothesis with a 99% likelihood of being present has a protein score contribution of 2.

6. The system of claim 5, wherein said processor is further arranged in (v) to calculate a protein score for each protein in the list by summing protein score contributions of peptide hypotheses present in said protein.

## Patentansprüche

1. Verfahren zur Identifizierung von Proteinen, die in einer Probe vorliegen, wobei das Verfahren umfasst:
(a) Durchführen eines Massenspektrometrie-Scans an dieser Probe, um ein Massenspektrum (130) zur Identifikation von Massen einer Vielzahl von Peptiden zu produzieren;
(b) Durchführen von MS/MS-Scans an dieser Vielzahl von Peptiden, um eine Vielzahl experimenteller MS/MS-Spektra zu produzieren;
(c) Vergleichen der experimentellen MS/MS-Spektra mit theoretischen Massenspektra, die aus einer Proteindatenbank stammen;
(d) Bereitstellen vielfacher Peptidhypothesen für jedes experimentelle MS/MS-Spektrum;
(e) Assoziieren eines Proteinscore-Beitrags mit jeder Peptidhypothese, wobei dieser Proteinscore-Beitrag auf einer Konfidenz basiert, dass dieses Peptid in dieser Probe vorliegt;
(f) Bestimmen einer Liste möglicher Proteine auf Basis dieser Peptidhypothesen;
(g) Für jedes Protein in dieser Liste, Berechnen eines Proteinscores auf Basis von Proteinscore-Beiträgen aus den Peptidhypothesen, die in diesem Protein enthalten sind;
(h) Identifizieren eines Gewinner-Proteins auf Basis dieser Proteinscores;
(i) Verrechnen der experimentellen MS/MS-Spektra der Peptidhypothesen, die in diesem Gewinner-Protein enthalten sind; und
(j) Wiederholen der Schritte (f) bis (i), wobei die Wiederholungen von Schritt (g) Berechnen eines ungenutzten Proteinscores für jedes Protein in der Liste einschließen, wobei dieser ungenutzte Proteinscore auf den Proteinscore-Beiträgen aus den in diesem Protein vorliegenden Peptidhypothesen basiert, aber nicht alle Proteinscore-Beiträge aus den Peptidhypothesen auf Basis von Spektra einschließt, die zuvor in Schritt (i) verrechnet wurden.

2. Verfahren gemäß Anspruch 1, wobei der Proteinscore-Beitrag einer Peptidhypothese als ein negativer Logarithmus von Eins minus der Wahrscheinlichkeit in Prozent, dass das Peptid in der Probe vorliegt, geteilt durch Einhundert berechnet wird, so dass beispielsweise eine Peptidhypothese, die mit einer Wahrscheinlichkeit von 99 % vorliegt, einen Proteinscore-Beitrag von 2 hat.

3. Verfahren gemäß Anspruch 2, wobei Berechnen eines Proteinscores für jedes Protein in der Liste Aufsummieren der Proteinscore-Beiträge der Peptidhypothesen, die in diesem Protein vorliegen, umfasst.

4. System zur Identifizierung von Proteinen, die in einer Probe vorliegen, wobei das System umfasst:
ein Massenspektrometer, hergerichtet zur Durchführung eines Massenspektrometrie-Scans an dieser Probe, um ein Massenspektrum (130) zur Identifikation von Massen einer Vielzahl von Peptiden zu produzieren, und zur Durchführen von MS/MS-Scans an dieser Vielzahl von Peptiden, um eine Vielzahl experimenteller MS/MS-Spektra zu produzieren;
einen Prozessor, hergerichtet zum:
(i) Vergleichen der experimentellen MS/MS-Spektra mit theoretischen Massenspektra, die aus einer Proteindatenbank stammen;
(ii) Bereitstellen vielfacher Peptidhypothesen für jedes experimentelle MS/MS-Spektrum;
(iii) Assoziieren eines Konfidenzwertes für jede Peptidhypothese;
(iv) Bestimmen einer Liste möglicher Proteine auf Basis dieser Peptidhypothesen;
(v) Für jedes Protein in dieser Liste, Berechnen eines Proteinscores auf Basis von Proteinscore-Beiträgen aus den Peptidhypothesen, die in diesem Protein enthalten sind;
(vi) Identifizieren eines Gewinner-Proteins auf Basis dieser Proteinscores;
(vii) Verrechnen der experimentellen MS/MS-Spektra der Peptidhypothesen, die in diesem Gewinner-Protein enthalten sind; und
(viii) Wiederholen der Schritte (iv) bis (vii), wobei die Wiederholungen von Schritt (v) Berechnen eines ungenutzten Proteinscores für jedes Protein in der Liste einschließen, wobei dieser ungenutzte Proteinscore auf den Proteinscore-Beiträgen aus den in diesem Protein vorliegenden Peptidhypothesen basiert, aber nicht alle Proteinscore-Beiträge aus den Peptidhypothesen auf Basis von Spektra einschließt, die zuvor in Schritt (vii) verrechnet wurden.

5. System gemäß Anspruch 4, wobei der Proteinscore-Beitrag einer Peptidhypothese als ein negativer Logarithmus von Eins minus der Wahrscheinlichkeit in Prozent, dass das Peptid in der Probe vorliegt, geteilt durch Einhundert berechnet wird, so dass beispielsweise eine Peptidhypothese, die mit einer Wahrscheinlichkeit von 99 % vorliegt, einen Proteinscore-Beitrag von 2 hat.

6. System gemäß Anspruch 5, wobei dieser Prozessor ferner zum (v) Berechnen eines Proteinscores für jedes Protein in der Liste durch Aufsummieren der Proteinscore-Beiträge der Peptidhypothesen, die in diesem Protein vorliegen, hergerichtet ist.

## Revendications

1. Procédé destiné à identifier des protéines présentes dans un échantillon, ledit procédé comprenant les étapes consistant à :
(a) exécuter un balayage par spectrométrie de masse sur ledit échantillon de façon à produire un spectre de masse (130) qui identifie les masses d'une pluralité de peptides ;
(b) exécuter des balayages SM / SM sur ladite pluralité de peptides de façon à produire une pluralité de spectres SM / SM expérimentaux ;
(c) comparer les spectres SM / SM expérimentaux à des spectres de masse théoriques obtenus à partir d'une base de données de protéines ;
(d) émettre plusieurs hypothèses de peptides pour chaque spectre SM / SM expérimental ;
(e) associer une contribution d'indice de pertinence de protéine à chaque hypothèse de peptide, ladite contribution d'indice de pertinence de protéine étant basée sur la confiance selon laquelle ledit peptide se trouve dans ledit échantillon ;
(f) déterminer une liste de protéines possibles sur la base desdites hypothèses de peptide ;
(g) pour chaque protéine dans ladite liste, calculer un indice de pertinence de protéine sur la base des contributions d'indice de pertinence de protéine à partir des hypothèses de peptide inclus dans ladite protéine ;
(h) identifier une protéine gagnante sur la base desdits indices de pertinence de protéine ;
(i) consommer les spectres SM / SM expérimentaux des hypothèses de peptide inclus dans ladite protéine gagnante ; et
(j) répéter les étapes (f) à (i), grâce à quoi les répétitions de l'étape (g) impliquent une étape consistant à calculer un indice de pertinence de protéine inutilisée pour chaque protéine dans la liste, ledit indice de pertinence de protéine inutilisée étant basé sur des contributions d'indice de pertinence de protéine à partir des hypothèses de peptide présent dans ladite protéine, mais ne comprenant aucune contribution d'indice de pertinence de protéine à partir des hypothèses de peptide sur la base des spectres qui ont été consommés précédemment dans l'étape (i).

2. Procédé selon la revendication 1, dans lequel la contribution d'indice de pertinence de protéine d'une hypothèse de peptide, est calculée comme étant le logarithme négatif de un moins la probabilité en pour cent que le peptide est présent dans l'échantillon divisé par cent, de telle sorte que, par exemple, une hypothèse de peptide qui présente une probabilité de 99 % d'être présent, présente une contribution d'indice de pertinence de protéine égale à 2.

3. Procédé selon la revendication 2, dans lequel l'étape consistant à calculer un indice de pertinence de protéine pour chaque protéine dans la liste, comprend une étape consistant à additionner les contributions d'indice de pertinence de protéine des hypothèses de peptide présent dans ladite protéine.

4. System destiné à identifier des protéines présentes dans un échantillon, ledit système comprenant :
un spectromètre de masse agencé de façon à exécuter un balayage par spectrométrie de masse sur ledit échantillon de manière à produire un spectre de masse (130) qui identifie les masses d'une pluralité de peptides, et est agencé de façon à exécuter des balayages SM / SM sur ladite pluralité de peptides de manière à produire une pluralité de spectres SM / SM expérimentaux ;
un processeur agencé de façon à :
(i) comparer les spectres SM / SM expérimentaux aux spectres de masse théoriques obtenus à partir d'une base de données de protéines ;
(ii) émettre plusieurs hypothèses de peptide pour chaque spectre SM / SM expérimental ;
(iii) associer une valeur de confiance à chaque hypothèse de peptide ;
(iv) déterminer une liste de protéines possibles sur la base desdites hypothèses de peptide ;
(v) pour chaque protéine dans ladite liste, calculer un indice de pertinence de protéine sur la base des contributions d'indice de pertinence de protéine à partir des hypothèses de peptide inclus dans ladite protéine ;
(vi) identifier une protéine gagnante sur la base desdits indices de pertinence de protéine ;
(vii) consommer les spectres SM / SM expérimentaux des hypothèses de peptide inclus dans ladite protéine gagnante ; et
(viii) répéter les étapes (iv) à (vii), grâce à quoi les répétitions de l'étape (v) impliquent une étape consistant à calculer un indice de pertinence de protéine inutilisée pour chaque protéine dans la liste, ledit indice de pertinence de protéine inutilisée étant basé sur des contributions d'indice de pertinence de protéine à partir des hypothèses de peptide présent dans ladite protéine, mais ne comprenant aucune contribution d'indice de pertinence de protéine à partir des hypothèses de peptide sur la base des spectres qui ont été consommés précédemment dans l'étape (vii).

5. Système selon la revendication 4, dans lequel la contribution d'indice de pertinence de protéine d'une hypothèse de peptide, est calculée comme étant le logarithme négatif de un moins la probabilité en pour cent que le peptide est présent dans l'échantillon divisé par cent, de telle sorte que, par exemple, une hypothèse de peptide qui présente une probabilité de 99 % d'être présent, présente une contribution d'indice de pertinence de protéine égale à 2.

6. Système selon la revendication 5, dans lequel ledit processeur est agencé en outre dans l'étape (v) de façon à calculer un indice de pertinence de protéine pour chaque protéine dans la liste, en additionnant les contributions d'indice de pertinence de protéine des hypothèses de peptide présent dans ladite protéine.
